# EUROPÄISCHE PATENTSCHRIFT

(11) **EP 3 595 804 B1**
(45) Veröffentlichungstag und Bekanntmachung des Hinweises auf die Patenterteilung: **19.08.2026**
(21) Anmeldenummer: 18711475.6
(22) Anmeldetag: 12.03.2018
(51) Int. Cl.: C08L 79/02

(54) **ORGANISCHE POLYMERPARTIKEL ENTHALTEND POLY(OXAZOLIN)-STABILISATOREN UND VERWENDUNG VON POLY(OXAZOLINEN) ZUR STABILISIERUNG VON ORGANISCHEN POLYMERPARTIKELN**
ORGANIC POLYMER PARTICLES CONTAINING POLY(OXAZOLINE) STABILIZERS AND USE OF POLY(OXAZOLINES) FOR STABILIZING ORGANIC POLYMER PARTICLES
PARTICULES POLYMÈRES ORGANIQUES CONTENANT COMME STABILISANT DE LA POLY(OXAZOLINE) ET UTILISATION DE POLY(OXAZOLINES) POUR LA STABILISATION DE PARTICULES POLYMÈRES ORGANIQUES

(30) Priorität: 14.03.2017 DE 102017002454
(43) Veröffentlichungstag der Anmeldung: 22.01.2020
(73) Patentinhaber: Friedrich-Schiller-Universität Jena, 07743 Jena (DE)
(72) Erfinder: LEISKE, Meike, Nicole, 27749 Delmenhost (DE); TRÄGER, Anja, 95367 Trebgast (DE); SCHUBERT, Ulrich, Sigmar, 07743 Jena (DE)
(74) Vertreter: Ackermann, Joachim
(86) Internationale Anmeldenummer: PCT/EP2018/000094
(87) Internationale Veröffentlichungsnummer: WO 2018/166651

(56) Entgegenhaltungen:
- EP-A1- 0 152 551
- EP-A1- 0 419 793
- WO-A1-2016/087598
- US-A- 5 837 768
- US-A1- 2008 131 395
- FLORIAN MANZENRIEDER ET AL: "Stabilization of Virus-like Particles with Poly(2-oxazoline)s", ANGEWANDTE CHEMIE, WILEY - V C H VERLAG GMBH & CO. KGAA, DE, vol. 123, no. 11, 20 January 2011 (2011-01-20), pages 2649 - 2653, XP071348621, ISSN: 0044-8249, DOI: 10.1002/ANGE.201006134

## Beschreibung

Die Erfindung betrifft das Gebiet der Herstellung und Verarbeitung von Polymerdispersionen, vorzugsweise der Polymerdispersionen aus nanopartikulären Partikeln, sowie die durch Verarbeitung von Polymerdispersionen erhaltenen Polymerpulver. Insbesondere betrifft die Erfindung die Herstellung und Verarbeitung von mit Poly(oxazolinen) stabilisierten organischen Polymerpartikeln, die vorzugsweise in flüssigem Wasser oder in mit Wasser mischbaren Flüssigkeiten verteilt vorliegen oder die durch Flüssigkeitsentzug aus diesen Flüssigkeiten gewonnen wurden. Dieses kann vorzugsweise durch Gefriertrocknung (Lyophilisierung) erfolgen.

Es ist bekannt, Polymerdispersionen durch grenzflächenaktive Stoffe, wie Tenside oder Schutzkolloide zu stabilisieren. Polymerdispersionen können durch ausgewählte Polymerisationsverfahren, wie durch Emulsionspolymerisation oder durch Suspensionspolymerisation, direkt aus den Monomeren erzeugt werden oder auch durch Dispergieren eines Polymers in einem Dispersionsmittel, wie Wasser und/oder einer mit Wasser mischbaren Flüssigkeit. Zur Herstellung dieser sogenannten Sekundärdispersionen werden Polymerpartikel in einem Dispergiermedium erhalten. Dies kann z. B. durch Ausfällen einer Polymerlösung durch Wasserzugabe oder durch die fortschreitende Dispergierung von Wasser in einem Polymer bis zum Auftreten einer Phaseninversion oder durch Dispergieren eines gelösten Polymeren durch Energieeintrag, beispielsweise durch Beschallen mit Ultraschall, erfolgen.

Aus dem Stand der Technik ist bekannt, dass bei der Herstellung von Polymerpartikeln zu deren Stabilisierung verschiedene Hilfsstoffe eingesetzt werden können. Dabei kann die Stabilisierung dieser Polymerpartikel in folgende Bereiche unterteilt werden:
i) Stabilisierung der Polymerpartikel während der Präparation und Aufreinigung von Polymerdispersionen (stabilisierende Hilfsstoffe häufig als "Surfactants" oder "Tenside" bezeichnet),
ii) Stabilisierung von Polymerpartikeln während der Lyophilisierung und Lagerung von Polymerdispersionen (stabilisierende Hilfsstoffe häufig als "Kryoprotectants" bezeichnet) und
iii) Stabilisierung von Polymerpartikeln während der Verwendung im Dispergiermedium, z.B. einem wässrigen System.

Bezüglich der Stabilisierung von Polymerpartikeln während der Präparation und Aufreinigung von Polymerdispersionen sind folgende Veröffentlichungen zu erwähnen:
C. Gomes *et al.* und H. Murakami *et al.* nutzten Poly(vinylalkohol) (PVA) um Poly-(DL-lactid-*co*-glycolid) (PLGA) Nanopartikel zu stabilisieren. Die Stabilisierung der Partikel während der Herstellung ist demnach bereits bekannt, jedoch werden zur Stabilisierung keine Poly(oxazolin)e eingesetzt. PVA hat zudem hämolytische Eigenschaften und sein Anteil muss vor einer biologischen Anwendung stark reduziert werden (J. Food Sci. 2011, 76, 16-24, Gomes, C., Moreira, R. G., Castell-Perez, E. Poly(DL-lactide-co-glycolide) (PLGA) Nanoparticles with entrapped trans-cinnamaldehyde and eugenol for antimicrobial delivery applications und Int. J. Pharm. 1999, 187, 143-152, Murakami, H., Kobayashi, M., Takeuchi, H. Kawashima, Y. Preparation of poly(DL-lactide-co-glycolide) nanoparticles by modified spontaneous emulsification solvent diffusion method).

In WO 2015/28685 A1 werden Poly(2-ethyl-2-oxazoline) als Stabilisator für Indomethacin während der Fällung dieses Stoffes genutzt, ohne dass dabei Polymerpartikel verwendet werden. Die Nutzung des Poly(oxazolin)s soll gewährleisten, dass die resultierenden Indomethacin-Partikel eine Größe <500 nm und eine verbesserte Löslichkeit in Wasser oder Medium besitzen, sowie dass diese Partikel redispergiert werden können. Eine Stabilisierung von Polymerpartikeln durch Poly(oxazolin)e wird nicht beschrieben.
C. Giardi *et al.* nutzen Lipid-poly(oxazolin) Copolymere, um daraus Mizellen zu bilden. Dabei wurde vorerst ein Lipidmakroinititator hergestellt, von dem aus die kationische Ringöffnungspolymerisation des 2-Methyl-2-oxazolins erfolgte. Es wurde einer Serie unterschiedlicher Copolymerer hergestellt, bei denen die Kettenlänge des Poly(2-oxazolin)s variiert wurde. Die synthetisierten Copolymere wurden dann in Hinblick auf die Oberflächenstabilisierung der entstandenen Mizellen untersucht. Die hergestellten Copolymere wurden für den Einsatz als polymere Tenside vorgeschlagen. Hier wurde ein eher amphiphiles Blockcopolymer verwendet mit einem wasserunlöslidchen Block (Lipid). Eine Stabilisierung von Polymerpartikeln durch Poly(oxazolin)e wird nicht beschrieben *(*React. Func. Polym. 2009, 69, 643-649, Giardi, C., Lapinte, V., Charnay, C., Robin, J. Nonionic poly(oxazolin)e surfactants based on renewable source: Synthesis, surface and bulk properties).
M. Miyamoto *et al.* stellten Blockcopolymere bestehend aus Poly(2-methyl-2-oxazolin) und Poly(propylenoxid) her, um deren Eignung als mögliche nichtionische Tenside zu prüfen. Die Oberflächenspannung der Polymere wurde gemessen und mit Pluronic^{®} F68 verglichen. Die gemessene Oberflächenaktivität der Copolymere nahm mit ansteigendem hydrophilem Polymeranteil ab. Eine Stabilisierung von Polymerpartikeln durch Poly(oxazolin)e wird nicht beschrieben *(*Polymer J., 1992, 24, 405-409, Miyamoto, M., Aoi, K., Yamanaka, H., Saegusa, T. Preparation of block copolymer consiting of poly(2-methyl-2-oxazoline) and poly(propylene oxide) blocks. A new nonionic surfactant). Aus diesem Dokument leitet der Fachmann ab, dass hydrophile Poly(oxazolin)e sich eigentlich nicht als Tenside eignen.

S. Kobayashi und H. Uyama nutzten Copolymere, welche aus einem hydrophoben (Fettsäure) und einem hydrophilen (Poly(2-ethyl-2-oxazolin) oder Poly(2-methyl-2-oxazolin)) Segment bestehen, um nichtionische Tenside herzustellen. Um die Tensideigenschaften zu bestimmen, wurden Messungen der Oberflächenspannung durchgeführt. Hier wurde ein eher amphiphiles Blockcopolymer mit einem wasserunlöslichen Fettsäure-Block verwendet. Eine Stabilisierung von feinteiligen Polymeren durch Poly(oxazolin)e wird nicht beschrieben *(*Macromolecules 1991, 24, 5473-5475, Kobayashi, S., Uyama, H. Synthesis of a nonionic polymeric surfactant from 2-oxazoline having a carboxylate component as the hydrophobic group).

Z. Amjad & D. Morgan nutzten Poly(2-ethyl-2-oxazolin) als Tensid zur Stabilisierung von Hydroxyapatit in wässriger Lösung. Eine Stabilisierung von Polymerpartikeln wird nicht offenbart *(*Phosphorus Research Bulletin 2011, 25, 33-38. Amjad, Z.; Morgan, D. Efficacy of hydroxyapatite dispersants in the presence of surfactants.)

Bezüglich der Stabilisierung von Polymerpartikeln während der Lyophilisierung und Lagerung von Polymerdispersionen sind folgende Veröffentlichungen zu erwähnen:
US 2014/0158931 A1 beschreibt Poly(2-oxazolin) als Stabilisator für metall-basierte Nanopartikel. Dabei werden die fertigen Metallpartikel in wässriger Lösung suspendiert und u.U. lyophilisiert. Die dabei Verwendung findenden Poly(2-ethyl-2-oxazolin)e haben eine beschriebene molare Masse (Mₙ) von 50,000 g mol⁻¹. Eine Stabilisierung von organischen Polymerpartikeln wird nicht offenbart.

CN 102552146 B beschreibt die Präparation und Lagerung von liposomalem Doxorubizin und Epirubizin. Hierbei werden die Liposome aus Phospholipiden aufgebaut und enthalten Tenside oder Kryoprotectants. Als Kryorotectant wird in diesem Zusammenhang unter anderem Poly(2-ethyl-2-oxazolin) mit einem Gesamtgehalt von bis zu 60% angegeben. Eine Stabilisierung von organischen Polymerpartikeln wird nicht offenbart.

A.T. Press *et al.* nutzten Polyvinylalkohol zur Partikelstabilisierung während der Lyophilisierung, indem den Partikeln zuvor 1 µg des Polymers zugegeben wurde. Die Stabilisierung von Partikeln während der Lyophilisierung ist demnach bereits bekannt, jedoch wird hier Polyvinylalkohol als Kryoprotectant eingesetzt (Nat. Commun. 2014, (5), 5565-5578. Press, A. T., Traeger, A., Pietsch, C., Mosig, A., Wagner, M., Clemens, M. G., Jbeily, N., Koch, N., Gottschaldt, M., Bézière, N., Ermolayev, V., Ntziachristos, V., Popp, J., Kessels, M. M., Qualmann, B., Schubert, U. S., Bauer, M. Cell type-specific delivery of short interfering RNAs by dyefunctionalised theranostic nanoparticles).

P. Fonte *et al.* nutzten Trehalose, Glukose, Sukrose, Fruktose oder Sorbitol mit einer Konzentration von 10 w/v% als Zusatz der PLGA Nanopartikel, um diese zu lyophiliseren. Die Stabilisierung der Partikel während der Lyophilisierung ist demnach bereits bekannt, jedoch werden als Kryoprotectants ausgewählte Kohlehydrate eingesetzt (Biomacromolecules, 2014, 15, 3753-3765, Fonte, P., Soared, S., Sousa, F., Costa, A., Seabra, V., Reis, S., Sarmento, B. Stability study perspective of the effect of freeze-drying using cryoprotectants on the structure of insulin loaded into PLGA nanoparticles).

Bezüglich der Stabilisierung von Polymerpartikeln während der Verwendung im Dispergiermedium sind zum Stand der Technik folgende Veröffentlichungen zu erwähnen:
D. Kedracki *et al.* nutzten bereits modifizierte hydrophobe Poly(2-oxazolin)e zur kovalenten DNA Bindung. Dafür wurden die ursprünglichen Doppelbindungen der Monomereinheiten teilweise über eine Thiol-En-Reaktion zu Aminogrupppen umgesetzt. Die dabei entstandenen Copolymere wurden benutzt, um DNA direkt an das Polymer zu binden und über den hydrophoben Seitenketten des Copolymers einen Tensidcharakter zu erzielen. Die Verwendung von wasserlöslichen Poly(oxazolin)en sowie eine Stabilisierung von organischen Polymerpartikeln wird nicht offenbart *(*Adv. Func. Mater. 2013, 24, 1133-1139. Kedracki, D.; Maroni, P.; Schlaad, H. Vebert-Nardin, C. Polymer-aptamer hybrid emulsion templating yields bioresponsive nanocapsules).

WO 2016/087598 A1 offenbart Mikrokapseln mit guter Lagerstabilität. Diese bestehen aus einem Kernmaterial sowie aus einer inneren Hüllschicht aus Polyvinylalkohol und aus einer äußeren Hüllschicht aus Poly(oxazolin). Als Kernmaterialien werden wasserunlösliche Stoffe genannt, darunter auch als Polyester benannte Verbindungen. Aus der Beschreibung geht hervor, dass damit Ester von Polycarbonsäuren mit C₄-C₃₀-Alkoholen und keine organischen Polymere gemeint sind. Dieses Dokument offenbart also lagerstabile Mikrokapseln aus wasserunlöslichen Kernmaterialien, die von Schichten aus Polyvinylalkohol und Poly(oxazolin) umhüllt sind.

Die im Stand der Technik beschriebenen Poly(oxazolin)-Tenside bestehen größtenteils aus Copolymeren, welche eine hydrophile Struktureinheit, nämlich die von der Polymerisation von Oxazolin abgeleitete Struktureinheit, und eine hydrophobe Struktureinheit, nämlich die von der Polymerisation hydrophober Monomerer abgeleitete Struktureinheit, beinhalten. Durch die Kombination dieser Struktureinheiten in einem Molekül können sowohl Interaktionen mit dem hydrophoben Polymerpartikel als auch mit dem hydrophilen Dispergiermedium gewährleistet werden. Zusätzlich können die Poly(oxazolin)-Tenside in wässrigem Medium eigene Nano- oder Mikrostrukturen ausbilden. Lediglich für die Herstellung von Indomethacin-Partikeln, einer niedermolekularen Substanz, in wässriger Lösung wurde bislang Poly(2-ethyl-2-oxazolin)-Homopolymer als Stabilisator vorgeschlagen. Polymerbasierte Partikelsysteme, z.B. PLGA, werden in der Regel durch Zugabe von Polyvinylalkohol bei der Präparation und Aufreinigung stabilisiert. Polyvinylalkohol zeigt jedoch Zytotoxizität und führt demzufolge dazu, dass hergestellte Partikel nachträglich aufgereigt werden müssen, z.B. durch Zentrifugation. Die Stabilisierung von organischen Polymerpartikeln, wie von polymerbasierten Nano- oder Mikropartikeln, mithilfe von wasserlöslichen Poly(oxazolin)en, wie Poly(2-ethyl-2-oxazolin) oder Poly(2-methyl-2-oxazolin), als Tensiden ist bisher nicht beschrieben worden. Bei diesen Poly(oxazolin)en handelt es sich um hydrophile Polymere.

Des Weiteren ist die Nutzung von Agentien zur Oberflächenstabilisierung und zum Strukturerhalt nach der Lyophilisierung (Kryoprotectants) grundsätzlich bekannt. Standardmäßig benutzte Agentien sind dabei Zucker (z.B. Trehalose, Glukose oder Saccharose), welche in vergleichsweisen großen Mengen von ca. 5-10 v/w% vor der Lyophilisierung zugegeben werden. Der Einsatz dieser Zuckers führt zu einer sehr guten Resuspendierung der Partikel. Allerdings können Aufnahmemechanismen dieser Partikel durch die Interaktion mit Zuckertransportern (GLUT-Transporter) auf den Zelloberflächen beeinflusst bzw. verändert werden. Außerdem sind Zucker hygroskopisch und können zu Problemen bei der Lagerung der erzeugten Partikel führen. Weiterhin sind Zucker osmotisch aktiv, was häufig für biologische Systeme ein unerwünschter Faktor ist. Die Stabilisierung von organischen Polymerpartikeln, insbesondere von Nanopartikeln, während der Lyophilisierung mittels Polyvinylalkohol ist ebenfalls möglich. Dieses kann jedoch zu erhöhter Zytotoxizität führen.

Die Stabilisierung von organischen Polymerpartikeln, insbesondere von Nano- oder Mikropartikeln, mithilfe von Poly(oxazolinen), wie Poly(2-ethyl-2-oxazolin) oder Poly(2-methyl-2-oxazolin) als Kryoprotectants ist bisher nicht beschrieben worden.

Eine Aufgabe der vorliegenden Erfindung bestand in der Bereitstellung von Partikeln enthaltend ausgewählte organische Polymere, die in hydrophilen Flüssigkeiten dispergiert vorliegen können und deren Dispersionen sich durch eine hervorragende Stabilität auszeichnen.

Eine weitere Aufgabe der vorliegenden Erfindung bestand in der Bereitstellung von Partikeln ausgewählter organischer Polymere, die in Pulverform vorliegen können und die sich durch eine hervorragende Stabilität auszeichnen.

Eine weitere Aufgabe der vorliegenden Erfindung bestand in der Bereitstellung Dispersionen von ausgewählten organischen Polymerpartikeln in hydrophilen Flüssigkeiten, die einfach und ohne nennenswerte Veränderung der Teilchengröße aufgereinigt werden können und/oder aufgearbeitet werden können, beispielsweise durch Gefriertrocknung oder durch Zentrifugation oder durch Filtration.

Eine weitere Aufgabe der vorliegenden Erfindung bestand in der Bereitstellung von Kryoprotectants für ausgewählte organische Polymerpartikel, die in hydrophilen Flüssigkeiten dispergiert vorliegen.

Die vorliegende Erfindung betrifft Zusammensetzungen enthaltend wasserlösliches Poly(oxazolin) und organische Polymerpartikel ausgewählt aus der Gruppe der Polymeren nach Anspruch 1 oder aus einem Gemisch von zwei oder mehreren dieser Polymere.

Im Rahmen der vorliegenden Beschreibung sind unter "hydrophilen Flüssigkeiten" Wasser und/oder mit Wasser mischbare Flüssigkeiten, wie Alkohle mit ein bis vier Kohlenstoffatomen oder Ketone mit zwei bis sechs Kohlenstoffatomen, zu verstehen. Im Rahmen der vorliegenden Beschreibung sind unter "Polymerpartikel" in Teilchenform vorliegende Polymere der oben genannten Stoffgruppen zu verstehen. Die Teilchen können in flüssiger Form in einer hydrophilen Flüssigkeit dispergiert vorliegen oder die Teilchen liegen vorzugsweise in fester Form vor, entweder in einer hydrophilen Flüssigkeit dispergiert oder in Form eines Pulvers. Die Größe der Partikel kann mittels visueller Methoden, beispielsweise durch Mikroskopie, ermittelt werden; bei Teilchengrößen im Nanobereich können Lichtstreuung oder Elektronenmikroskopie herangezogen werden. Die Gestalt der Polymerteilchen kann beliebig sein, beispielsweise sphärisch, ellipsoid oder irrgulär. Die Polymerteilchen können auch Aggregate aus mehreren Primärteilchen bilden. Vorzugsweise liegen die Partikel aus organischen Polymeren in der Form von Nanopartikeln vor. Die Teilchen können neben den organischen Polymeren noch weitere Bestandteile enthalten, beispielsweise Wirkstoffe oder Hilfs- oder Zusatzstoffe.

Die Begriffe "Teilchen" oder "Partikel" werden im Rahmen der vorliegenden Beschreibung synonym verwendet.

Unter "Nanopartikeln" sind im Rahmen der vorliegenden Beschreibung Teilchen zu verstehen, deren Durchmesser kleiner als 1 µm ist und die aus einem oder mehreren Molekülen aufgebaut sein können. Sie zeichnen sich allgemein durch ein sehr hohes Oberflächen-zu-Volumen-Verhältnis aus und bieten damit eine sehr hohe chemische Reaktivität. Nanopartikel können aus Polymeren bestehen oder enthalten neben den Polymeren noch andere Bestandteile, wie z.B. Wirkstoffe oder Hilfs- oder Zusatzstoffe.

Unter "Polymeren" sind im Rahmen der vorliegenden Beschreibung die oben genannten organischen Verbindungen zu verstehen, die durch Wiederholung von bestimmten Einheiten (Monomereinheiten oder Wiederholungseinheiten) gekennzeichnet sind. Polymere können aus einer Art oder aus mehreren Arten verschiedener Wiederholungseinheiten bestehen. Polymere werden durch die chemische Reaktion von Monomeren unter Ausbildung von kovalenten Bindungen hergestellt (Polymerisation) und bilden durch Verknüpfen der polymerisierten Einheiten das sogenannte Polymerrückgrad. Dieses kann Seitenketten aufweisen, an denen sich funktionelle Gruppen befinden können. Besitzen Polymere zum Teil hydrophobe Eigenschaften, können sie in wässriger Umgebung nanoskalige Strukturen (z.B. Nanopartikel, Mizellen, Vesikel) ausbilden. Homopolymere bestehen nur aus einer Monomereinheit. Copolymere bestehen hingegen aus mindestens zwei unterschiedlichen Monomereinheiten, welche statistisch, als Gradient, alternierend oder als Block angeordnet sein können.

Unter "Kryoprotectants" sind im Rahmen der vorliegenden Beschreibung wasserlösliche Stoffe oder Stoffgemische zu verstehen, die der Stabilisierung eines Partikels während der Lyophilisierung (Gefriertrocknung) dienen. Sie können dem im Dispersionsmedium dispergierten Partikeln, z.B. der Partikelsuspension, bereits während der Herstellung oder im Nachhinein (aber vor der Lyophilisierung) zugegeben werden. Des Weiteren sind diese Stoffe oder Stoffgemische nicht kovalent an den Partikel gebunden.

Unter "Tensiden" oder "Surfactants" sind im Rahmen der vorliegenden Beschreibung wasserlösliche Stoffe oder Stoffgemische zu verstehen, die der Stabilisierung von Partikeln während der Herstellung und der Lagerung in wässrigen Medien dienen. Sie werden üblicherweise dem Dispergiermedium, z.B. der wässrigen Phase, bei der Herstellung der Partikel zugegeben, können aber auch nach deren Herstellung zur Stabilisierung der erhaltenen Dispersion zugegeben werden.

Unter "wasserlöslichen Verbindungen" oder "wasserlöslichen Poly(oxazaolin)en" sind im Rahmen der vorliegenden Beschreibung Verbindungen bzw. Poly(oxazolin)e zu verstehen, die sich zu mindestens 50 g/L Wasser bei 25 °C lösen.

Unter "Wirkstoffen" sind im Rahmen der vorliegenden Beschreibung Verbindungen oder Gemische von Verbindungen zu verstehen, die auf einen lebenden Organismus eine gewünschte Wirkung ausüben. Dabei kann es sich z.B. um pharmazeutische Wirkstoffe oder um agrochemische Wirkstoffe handeln. Wirkstoffe können nieder- oder hochmolekulare organische Verbindungen sein. Bevorzugt handelt es sich bei den Wirkstoffen um niedermolekulare pharmazeutisch wirksame Substanzen oder um höhermolekulare pharmazeutisch wirksame Substanzen, wobei inbesondere hydrophile Wirkstoffe aus potentiell therapeutisch nutzbaren Nukleinsäuren (z.B. short interferin RNA, short hairpin RNA, micro RNA, plasmid DNA) oder aus potentiell nutzbaren Proteinen (z.B. Antikörper, Interferone, Zytokine) eingesetzt werden können.

Unter dem Begriff "pharmazeutischer Wirkstoff" wird im Rahmen der vorliegenden Beschreibung jede(s) beliebige anorganische oder organische Molekül, Substanz oder Verbindung verstanden, das (die) eine pharmakologische Wirkung aufweist. Der Begriff "pharmazeutischer Wirkstoff" wird hierin mit dem Begriff "Arzneimittel" synonym verwendet.

Wirkstoffe können dabei solche sein, die ohne Einschluss in einen Nanopartikel oder ein Liposom lediglich eine geringe oder keine Bioverfügbarkeit haben, eine geringe bzw. keine Stabilität *in vivo* aufweisen oder nur in bestimmten Zellen eines Organismus wirken sollen.

Unter "Hilfs- und Zusatzstoffen" sind im Rahmen der vorliegenden Beschreibung Substanzen zu verstehen, die einer Formulierung zugesetzt werden, um dieser bestimmte zusätzliche Eigenschaften zu verleihen und/oder um deren Verarbeitung zu erleichtern. Beispiele für Hilfs- und Zusatzsstoffe sind Tracer, Kontrastmittel, Trägerstoffe, Füllstoffe, Pigmente, Farbstoffe, Parfums, Gleitmittel, UV-Stabilisatoren, Antioxidantien oder Tenside. Insbesondere ist unter "Hilfs-und Zusatzstoffen" jede pharmakologisch verträgliche und therapeutisch sinnvolle Substanz zu verstehen, die kein pharmazeutischer Wirkstoff ist, jedoch zusammen mit einem pharmazeutischen Wirkstoff in einer pharmazeutischen Zusammensetzung formuliert werden kann, um qualitative Eigenschaften der pharmazeutischen Zusammensetzung zu beeinflussen, insbesondere zu verbessern. Bevorzugt entfalten die Hilfs- und/oder Zusatzstoffe keine oder im Hinblick auf die beabsichtigte Behandlung keine nennenswerte oder zumindest keine unerwünschte pharmakologische Wirkung.

Bevorzugte erfindungsgemäße Zusammensetzungen betreffen eine pharmazeutische Zusammensetzung, die ein nanostrukturiertes Trägersystem gemäß der Erfindung, mindestens einen pharmazeutischen Wirkstoff sowie geeignete Hilfs- und Zusatzstoffe enthält.

Bevorzugte erfindungsgemäße Zusammensetzungen enthalten Partikel aus ausgewählten organischen Polymeren mit einem mittleren Durchmesser D₅₀ von weniger als 10 µm, insbesondere von weniger als 1 µm. Dabei bedeutet D₅₀, dass 50 Volumen-% der Partikel kleiner sind als der für D₅₀ angegebene Wert. Der D₅₀-Wert kann für die Zwecke der vorliegenden Beschreibung durch Lichtstreuung oder durch Mikroskopie, z.B. mittels Transmissions-Elektronenmikroskop oder mittels Raster-Elektronenmikroskop, bestimmt werden.

Besonders bevorzugte erfindungsgemäße Zusammensetzungen sind solche mit Partikeln ausgewählter organischer Polymerer mit Teilchendurchmessern im Bereich von 50 bis 999 nm. Die Teilchendurchmesser betreffen die Primärteilchen und können für die Zwecke der vorliegenden Beschreibung durch (dynamische) Lichtstreuung ((D)LS), durch Nanosize-Tracking-Analysis (NTA), oder durch Elektronenmikroskopie, z.B. mittels Transmissions-Elektronenmikroskop oder mittels Raster-Elektronenmikroskop bestimmt werden.

Die Partikel ausgewählter organischer Polymerer können in den erfindungsgemäßen Zusammensetzungen als Pulver in fester Form vorliegen oder sie können in hydrophilen Lösungsmitteln dispergiert vorliegen, wobei die Teilchen im Dispergiermedium in flüssiger Form oder insbesondere in fester Form vorliegen.

In bevorzugten erfindungsgemäßen Zusammensetzungen bilden die Partikel ausgewählter organischer Polymerer eine disperse Phase in einer Flüssigkeit enthaltend Wasser und/oder mit Wasser mischbare Verbindungen. In besonders bevorzugten erfindungsgemäßen Zusammensetzungen sind die Partikel ausgewählter organischer Polymerer in der hydrophilen Flüssigkeit dispergiert.

In einer weiteren bevorzugten Ausführungsform der erfindungsgemäßen Zusammensetzungen liegen die Teilchen des feinteiligen, ausgewählten organischen Polymers in fester Form vor und die Teilchen sind von dem Poly(oxazolin) umhüllt.

Bei dem erfindungsgemäß eingesetzten ausgewählten organischen Polymer handelt es sich um ein ausgewähltes Polymer gemäß der oben gegebenen Definition oder um ein Gemisch derartiger Polymere.

Stoffklassen, welche das Rückgrat des erfindungsgemäß eingesetzten Polymers bilden können, sind von organischen Dicarbonsäuren und organischen Diolen und/oder von organischen Hydroxycarbonsäuren abgeleitete Polyester, Polycarbonate, von Estern ethylenisch ungesättigter Carbonsäuren abgeleitete Polymere, wie Polyacrylat- oder Polymethacrylat.

Die erfindungsgemäß eingesetzten Polymere können als lineare Polymere vorliegen oder aber es kann sich um Pfropf-, Kamm- und Sternpolymere, Dendrimere, Leiterpolymere, ringförmige Polymere, Polycatenane und Polyrotaxane handeln.

Die Löslichkeit der erfindungsgemäß eingesetzen Polymere kann durch CoPolymerisation mit geeigneten Monomeren und/oder durch Funktionalisierung beeinflusst werden. Dem Fachmann sind solche Techniken bekannt.

Die erfindungsgemäß eingesetzten organischen Polymere können einen weiten Molmassenbereich umfassen. Typische Momassen (Mₙ) bewegen sich im Bereich von 2.000 bis 500.000 g/mol, insbesondere von 5.000 bis 49.900 g/mol. Diese Molmassen können durch ¹H-NMR-Spektroskopie des gelösten Polymers bestimmt werden. Insbesondere lassen sich zur Bestimmung der Molmassen eine analytische Ultrazentrifuge oder chromatographische Methoden, wie die Größenausschlusschromatographie, einsetzen.

Bevorzugt eingesetzte organische Polymere weisen eine mittlere Molmasse (Zahlenmittel) im Bereich von 5.000 bis 20.000 g/mol auf, ermittelt durch ¹H-NMR-Spektroskopie oder durch Verwendung einer analytischen Ultrazentrifuge.

Der Anteil des organischen Polymers in der erfindungsgemäßen Zusammensetzung kann einen weiten Bereich umfassen. Liegt das organische Polymer in einem Dispersionsmedium dispergiert vor, so beträgt dessen Anteil an der Gesamtzusammensetzung im Allgemeinen 0,5 bis 20 Gew.-%, vorzugsweise 1 bis 5 Gew.-%. Liegt das organische Polymer in Partikelform zusammen mit dem Poly(oxazolin) vor, beispielsweise als pulverförmiger Feststoff, so beträgt dessen Anteil an der Gesamtzusammensetzung im Allgemeinen 0,1 bis 30 Gew.-%, vorzugsweise 0,5 bis 10 Gew.-%.

Die Herstellung des erfindungsgemäß eingesetzen organischen Polymers kann mit den üblichen Polymerisationsverfahren erfolgen. Beispiele dafür sind die Polymerisation in Substanz, die Polymerisation in Lösung oder die Emulsions- bzw. Suspensionspolymerisation. Dem Fachmann sind diese Vorgehensweisen bekannt.

Zu den ganz besonders bevorzugt eingesetzten organischen Polymeren zählt die Gruppe der Polyester. Dabei handelt es sich in der Regel um Polykondensate abgeleitet von aliphatischen oder cycloaliphatischen Diolen und von aliphatischen, cycloaliphatischen und/oder aromatischen Dicarbonsäuren oder deren polyesterbildenden Derivaten, wie deren Dialkylestern. Es kann sich auch um Polykondensate handeln, die sich von aliphatischen oder cycloaliphatischen Hydroxymonocarbonsäuren oder deren polyesterbildenden Derivaten, wie Hydroxymonocarbonsäurealkylestern, ableiten.

Zu den bevorzugt eingesetzten Polyestern zählen die von aliphatischen Diolen und von aliphatischen Dicarbonsäuren oder von aliphatischen Dicarbonsäurealkylestern abgeleiteten Polyester und die von aliphatischen Hydroxymonocarbonsäuren oder von aliphatischen Hydroxymonocarbonsäurealkylestern abgeleiteten Polyester.

Besonders bevorzugt eingesetzt werden Polyhydroxyalkanoat(e), und davon ganz besonders bevorzugt Milchsäurehomo- (PLA) oder -copolymer(e). Ein ganz besonders bevorzugt eingesetzter Polyester ist das Milchsäure-Glycolsäure-Copolymer (PLGA).

Weitere ganz bevorzugte organische Polymere sind von Estern ethylenisch ungesättigter Carbonsäuren abgeleitete Polymere, insbesondere von Polyacrylsäureestern und/oder von Polymethacrylsäureestern abgeleitete Homo- oder Copolymere, ganz besonders bevorzugt Acrylsäurealkylester-Methylacrylsäurealkylester-Copolymere.

Die erfindungsgemäßen Zusammensetzungen enthalten ein oder mehrere wasserlösliche Poly(oxazolin)e. Die Menge an Poly(oxazoline)n, bezogen auf die Gesamtmenge der erfindungsgemäßen Zusammensetzung, beträgt in der Regel 0,1 bis 30 Gew.-%.

Poly(oxazolin)e sind bekannte Verbindungen. Diese werden üblicherweise durch kationische Ringöffnungspolymerisation von Oxazolinen, vorzugsweise von 2-Oxazolinen, in Lösung und in Gegenwart eines Initiators hergestellt. Beispiele für Initiatoren sind Elektrophile, wie Salze oder Ester von aromatischen Sulfonsäuren oder Carbonsäuren oder Salze oder Ester von aliphatischen Sulfonsäuren oder Carbonsäuren oder aromatische Halogenverbindungen. Es können auch mehrfachfunktionelle Elektrophile als Initiatoren eingesetzt werden. Dabei können neben linearen Poly(oxazolin)en auch verzweigte oder sternförmige Moleküle entstehen. Beispiele für bevorzugte Initiatoren sind Ester der Arylsulfonsäuren, wie Methyltosylat, Ester der Alkansulfonsäuren, wie Trifluormethansulfonsäure, oder Mono- oder Dibrombenzol. Die Polymerisation wird üblicherweise in einem polaren aprotischen Lösungsmittel durchgeführt, beispielsweise in Acetonitril.

Als Oxazoline zur Herstellung der erfindungsgemäß eingesetzten Poly(oxazolin)e werden 2-Oxazoline (4,5-Dihydrooxazole) mit einer C=N-Doppelbindung zwischen dem Kohlenstoffatom 2 und dem Stickstoffatom eingesetzt. Diese können am 2-, 4- und/oder 5-Kohlenstoffatom und/oder am 3-Stickstoffatom substituiert sein, vorzugsweise am 2-Kohlenstoffatom und/oder am 3-Stickstoffatom.

Bevorzugt werden 2-Oxazoline eingesetzt, welche an 2-Position einen Substituenten enthalten. Beispiele für solche Substituenten sind Methyl oder Ethyl.

Neben den 2-Oxazolinen können bei der Herstellung der erfindungsgemäß eingesetzten wasserlöslichen Poly(oxazolin)e noch geringe Mengen weiterer mit 2-Oxazolinen copolymerisierbarer Monomere eingesetzt werden.

Die erfindungsgemäß eingesetzten wasserlöslichen Poly-(oxazolin)e enthalten in der Regel mindestens 80 Gew.-%, insbesondere mindestens 90 Gew. % und ganz besonders bevorzugt mindestens 95 Gew. %, bezogen auf deren Gesamtmasse, an wiederkehrenden Struktureinheiten der Formel I und/oder der Formel II

-NR¹-CR³H-CR⁴H- (I),

-NR¹-CR³H-CR⁴H-CR⁵H- (II),

worin
R¹ einen Rest der Formel -CO-R² bedeutet,
R³, R⁴ und R⁵ unabhängig voneinander Wasserstoff, Methyl, Ethyl, Propyl oder Butyl bedeuten,
R² ausgewählt wird aus der Gruppe bestehend aus Wasserstoff, Methyl, Ethyl,
-CₘH₂ₘ-X oder -(CₙH₂ₙ-O)ₒ-(CₚH₂ₚ-O)_{q}-R⁶,
R⁶ Wasserstoff oder C₁-C₆-Alkyl, insbesondere Methyl oder ganz besonders bevorzugt Wasserstoff ist,
m eine ganze Zahl von 1 bis 6 ist,
X ausgewählt wird aus der Gruppe bestehend aus Hydroxyl, Alkoxy, Amino, N-Alkylamino, N,N-Dialkylamino, Carboxyl, Carbonsäureester, Sulfonyl,
Sulfonsäureester oder Carbamat,
n und p unabhängig voneinander ganze Zahlen von 2 bis 4 sind, wobei n ungleich p ist, n vorzugsweise 2 ist und p vorzugsweise 3 bedeutet, und
o und q unabhängig ganze Zahlen von 0 bis 60, insbesondere 1 bis 20 und ganz besonders bevorzugt 2 bis 10 sind, wobei mindestens eines der o oder q ungleich 0 ist.

Bevorzugte erfindungsgemäß eingesetzte wasserlösliche Poly(oxazolin)e sind solche, in denen R² Wasserstoff, Methyl oder Ethyl ist und R³ bis R⁵ Wasserstoff bedeuten oder in denen R² Wasserstoff, Methyl oder Ethyl ist und zwei der Reste R³ bis R⁵ Wasserstoff sind ist und einer der Reste R³ bis R⁵ Methyl oder Ethyl ist.

Das Molmassen der erfindungsgemäß eingesetzten Poly(oxazolin)e beträgt in der Regel 5.000 bis 500.000 g/mol, insbesondere 5.000 bis 20.000 g/mol. Die Molmasse wird für die Zwecke der vorliegenden Beschreibung durch ¹H-NMR-Analyse bestimmt.

Ganz besonders bevorzugte erfindungsgemäße Zusammensetzungen enthalten ein wasserlösliches Poly(oxazolin), das mindestens 90 Gew.-%, insbesondere mindestens 95 Gew.-%, bezogen auf dessen Gesamtmasse, an wiederkehrenden Struktureinheiten der Formel I aufweist, worin R² Methyl oder Ethyl bedeutet.

Weitere bevorzugte erfindungsgemäße Zusammensetzungen enthalten nebem dem feinteiligen organischen Polymer einen oder mehrere pharmazeutische Wirkstoffe.

Die erfindungsgemäßen Zusammensetzungen können durch Fällung, vorzugsweise durch Nanofällung hergestellt werden. Dazu werden die erfindungsgemäß verwendeten organischen Polymere, welche durch die Anwesenheit polarer Gruppen hydrophil sind, in einem nicht mit Wasser mischbaren Lösungsmittel, wie Dichlormethan, oder in einem mit Wasser mischbaren Lösungsmittel, wie Aceton oder Ethylacetat, gelöst. Diese Lösung wird in ein hydrophiles Dispergiermedium, welches ein wasserlösliches Poly(oxazolin) enthält, vorzugsweise in wasserlösliches Poly(2-oxazolin) enthaltendes Wasser oder in ein wasserlösliches Poly(2-oxazolin) enthaltendes Gemisch aus Wasser und Methanol oder Ethanol, eingetropft. Dieses erfolgt vorzugsweise unter starkem Rühren. Dadurch kann die Herstellung kleinerer Partikel gefördert werden. Das organische Polymer wird im Dispergiermedium in feinverteilter Form abgeschieden.

Alternativ können die erfindungsgemäßen Zusammensetzungen auch durch Emulgieren erzeugt werden, vorzugsweise durch Nanoemulsion. Dazu werden die erfindungsgemäß verwendeten organischen Polymere, welche durch die Anwesenheit polarer Gruppen hydrophil sind, in einem nicht mit Wasser mischbaren Lösungsmittel, wie Dichlormethan, oder in einem mit Wasser mischbaren Lösungsmittel, wie Aceton oder Ethylacetat, gelöst. Diese Lösung wird mit einem hydrophilen Dispergiermedium, welches ein wasserlösliches Poly(oxazolin) enthält, vorzugsweise mit wasserlöslisches Poly(2-oxazolin) enthaltenden Wasser oder mit wasserlösliches Poly(2-oxazolin) enthaltenden Gemisch aus Wasser und Methanol oder Ethanol, kombiniert, wodurch sich vorzugsweise zwei flüssige Phasen ausbilden. Anschließend wird dieses Gemisch durch Energieintrag emulgiert, vorzugsweise durch Beschallen mit Ultraschall.

Zusätzlich zu dem organischen Polymer können bei dessen Dispergierung im Dispergiermedium ein oder mehrere Wirkstoffe und/oder ein oder mehrere Hilfs- und Zusatzstoffe zugegen sein. Alternativ können diese Wirkstoffe und/oder Hilfs- und Zusatzstoffe nach dem Dispergieren des organischen Polymers in der hydrophilen Flüssigkeit hinzugefügt werden.

Üblicherweise hat man bei der Herstellung von in hydrophilen Flüssigkeiten dispergierten organischen Polymeren bislang Polyvinylalkohol oder Polyethlyenpolypropylen-Blockcopolymere, wie Pluronic ^{®} F127 oder Pluronic ^{®} F68, oder Polyoxyethylensorbitanmonooleat, wie Tween^{®} 80, eingesetzt. Die erfindungsgemäßen Zusammensetzungen zeichnen sich im Vergleich zu diesen bekannten Zusammensetzungen durch eine vergrößerte Stabilität aus.

Die Abtrennung der Polymerpartikel und des Poly(oxazolin)-Stabilisators aus der hydrophilen Flüssigkeit kann auf unterschiedliche Weisen erfolgen. Beispiele dafür sind Zentrifugation, Ultrafiltration oder Dialyse.

Besonders bevorzugt erfolgt die Aufarbeitung der Polymerpartikel, welche gegebenenfalls zusätzlich einen Wirkstoff und/oder Hilfs- und Zusatzstoffe enthalten können, und des Poly(oxazolin)-Stabilisators aus der hydrophilen Flüssigkeit durch Gefriertrocknung. Dabei hat sich überraschenderweise gezeigt, dass das Poly-(oxazolin)e als Kryoprotectoren wirken. Im Vergleich zu bislang eingesetzten Kryoprotectants auf Zuckerbasis, wie Hepes Buffered Glucose, Glukose oder Saccharose, ist bei den mit Poly(oxazolin) stabilisierten Dispersionen nach der Gefriertrocknung kein weiterer Aufarbeitungsschritt notwendig, beispielsweise durch Zentrifuieren, da durch die erfindungsgemäß erhaltenen feinteiligen Polymere keine Zytotoxizität, Hämolyse oder andere bekannte Immunreaktionen ausgelöst werden.

Die erfindungsgemäß hergestellte Polymerdispersion kann nach der Herstellung weiter aufgereinigt werden. Gängige Verfahren beinhalten das Reinigen mittels Dialyse, mittels Ultrafiltration, mittels Filtration oder mittels Zentrifugieren.

Durch Reinigung mittels Filtration können Teilchen, wie zum Beispiel Aggregate, aber auch Endotoxine, nicht verkapselte Wirkstoffe oder Hilfsstoffe oder bakterielle Verunreinigungen aus der Dispersion abgetrennt werden. Dabei kann sich die Partikelkonzentration ändern.

Durch Reinigung mittels Dialyse können Lösungsmittel oder gelöste Moleküle aus der Dispersion abgetrennt werden. Das Verfahren ist hinsichtich der dispergierten Partikel weitgehend unabhängig von der Partikelgröße. Allerdings verringert sich bei der Durchführung der Dialyse die Konzentration der dispergierten Teilchen und das Verfahren ist relativ zeitaufwendig.

Durch Reinigung mittels Zentrifugieren können ebenfalls Lösungsmittel oder gelöste Moleküle aus der Dispersion abgetrennt werden. Allerdings verringert sich auch bei diesem Verfahren die Konzentration der dispergierten Teilchen. Außerdem lassen sich nur Dispersionen mit Nanoteilchen größeren Durchmessers, z.B. von mehr als 150 nm, behandeln und die Teilchen können dabei in Mitleidenschaft gezogen werden. Ferner kann das Redispergieren der auf diese Weise gewonnenen Teilchen Schwierigkeiten bereiten.

Beim erfindungsgemäßen Einsatz der Poly(oxazolin)e bei der Aufreinigung von Polymerdispersionen hat sich überraschenderweise gezeigt, dass die bislang bekannten Nachteile bei der Dialyse, beim Zentrifugieren und bei der Reinigung mittels Filter nur in erheblich verringertem Ausmaß auftreten oder sogar ganz verschwunden sind.

Die Erfindung betrifft auch die Verwendung von wasserlöslichem Poly(oxazolin) zur Stabilisierung von organischen Polymerpartikeln ausgewählt aus der Gruppe der Polymere nach Anspruch 15 oder aus einem Gemisch von zwei oder mehreren dieser Polymere in einer Flüssigkeit enthaltend Wasser und/oder mit Wasser mischbare Verbindungen.

Des weiteren betrifft die Erfindung die Verwendung von wasserlöslichem Poly(oxazolin) zur Stabilisierung von organischen Polymerpartikeln ausgewählt aus der Gruppe der Polymere nach Anspruch 16 oder aus einem Gemisch von zwei oder mehreren dieser Polymere bei der Gefriertrocknung.

Dabei werden besonders bevorzugt Suspensionen des feinverteilten organischen Polymers in einer hydrophilen Flüssigkeit eingesetzt.

Außerdem betrifft die Erfindung die Verwendung wasserlöslichem Poly(oxazolin) als Stabilisatoren bei der Herstellung oder bei der Aufarbeitung von organischen Polymerpartikeln ausgewählt aus der Gruppe der Polymere nach Anspruch 17 oder aus einem Gemisch von zwei oder mehreren dieser Polymere in einer hydrophilen Flüssigkeit.

Dabei werden besonders bevorzugt die Aufarbeitungsverfahren Dialyse, Zentrifugieren oder Filtration eingesetzt.

Schließlich betrifft die Erfindung die Verwendung wasserlöslichem Poly(oxazolin) zur Stabilisierung von pulverförmigen organischen Polymerpartikeln ausgewählt aus der Gruppe der Polymere nach Anspruch 18 oder aus einem Gemisch von zwei oder mehreren dieser Polymere.

Die folgenden Beispiele erläutern die Erfindung ohne diese zu begrenzen.

### Beispiel 1: Herstellung von Poly(2-oxazolin)en (POₓ)

Die Synthese von Poly(2-oxazolin)en wurde bereits in der Literatur beschrieben (vergl. z.B. Wiesbrock, F. et al. Macromolecular Rapid Communications 2004, 25, 1895-1899). Die Vorgehensweise wird deshalb beispielhaft für Poly(2-ethyl-2-oxazolin) mit einem Polymerisationsgrad (DP) von 61 (P(EtOx)₆₁) beschrieben.

In einem Mikrowellenreaktionsgefäß wurden unter inerten Bedingungen 2-Ethyl--2-oxazolin (6,06 mL, 60,0 mmol), Methyltosylat (0,15 mL, 0,1 mmol) und Acetonitril (8,79 mL) gemischt. Das Reaktionsgefäß wurde dann in einer Synthesemikrowelle für 14 min auf 140 °C erhitzt. Anschließend wurde die Reaktion mittels der Zugabe von 0,5 mL deionisiertem Wasser terminiert und über Nacht bei Raumtemperatur gerührt. Die resultierende Lösung wurde aufgereinigt, indem sie mit Dichlormethan verdünnt und dann in einem Überschuss eiskaltem Diethylether gefällt wurde. Das ausgefallene Polymer wurde dann abfiltriert und in Dichlormethan gelöst. Das Lösungsmittel wurde anschließend am Rotationsverdampfer entfernt und das Polymer bis zur vollständigen Lösungsmittelfreiheit am Hochvakuum getrocknet. Das finale Produkt lag als kristalliner, weißer Feststoff vor.

¹H-NMR (CDCl₃, 300 MHz): δ = 4,34 (0,1H, s, backbone-OH), 3,44 (4,0H, s, backbone), 3,02 (0,3H, s, CH₃-backbone), 2,4 (1,7H, m, CH₂ (EtOx)), 1,11 (2,5H, s, CH₃ (EtOx)) ppm.

SEC (Eluent: DMAc¹⁾, 0,21% LiCl, PS²⁾-Standard): Mₙ = 11.200 g mol⁻¹,

M_{w} = 12.200 g mol⁻¹, D = 1,09.

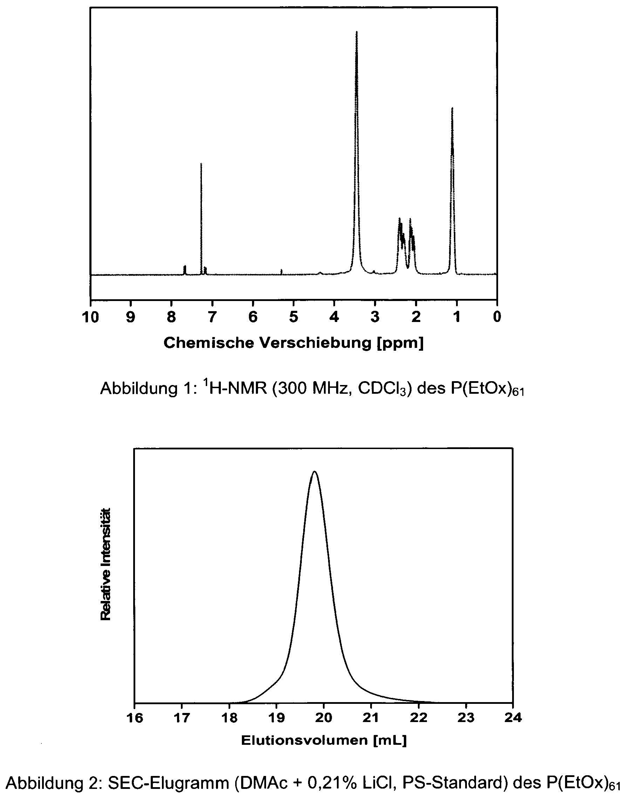

Abbildung 1 zeigt das ¹H-NMR (300 MHz, CDCl₃) Diagramm des P(EtOx)₆₁.

Abbildung 2 zeigt das Größenausschlusschromatographie-Diagramm (DMAc¹⁾, 0,21% LiCl, PS²⁾-Kalibration) des P(EtOx)₆₁.
1) DMAc = Dimethylacetamid
2) PS = Polystyrol

### Beispiel 2: Gefriertrocknungsexperimente

PLGA³⁾ Nanopartikel wurden mittels Nanofällung hergestellt (siehe Beispiel 3) und mit der dynamischen Lichtstreuung bezüglich Größe (Partikeldurchmesser, z-Mittel) und Größenverteilung (PDI) charakterisiert. Nach dieser Charakterisierung wurde der Nanopartikelsuspension eine bestimmte Menge an Kryoprotektor in verschiedenen Konzentrationen zugegeben. Die Suspensionen wurden in einem -80 °C Gefrierschrank gefroren und anschließend gefriergetrocknet (24 h, -56 °C, 0,01 mbar). Etwa 2 mg des resultierenden Pulvers wurden in 1 mL Reinstwasser wieder resuspendiert und dann erneut mit der dynamischen Lichtstreuung bezüglich Partikeldurchmesser (z-Mittel) und PDI charakterisiert. Die Verhältnisse wurden bestimmt, indem die Werte, welche nach der Lyophilisierung aufgenommen wurde, durch jene dividiert wurden, welche direkt nach der Präparation bestimmt wurden.

**Tabelle 1: Ergebnisse der Lyophilisierungsexperimente von PLGA³⁾ Nanopartikeln mit unterschiedlichen Konzentrationen P(EtOx)₆₁ als Kryoprotektor.**

| **Konzentration Kryoprotektor [%]** | **0** | **0,05** | **0,10** | **0,50** | **1,00** | **2,50** | **5,00** |
|---|---|---|---|---|---|---|---|
| Größe nach Präparation [nm] | 88,9 ± 0,8 | | | | | | |
| Größe nach Lyophilisierung [nm] | 2131,2 ± 1090,4 | 171,2 ± 12,7 | 130,5 ± 19,1 | 123,5 ± 24,3 | 133,4 ± 28,6 | 129,8 ± 20,1 | 138,5 ± 31,6 |
| Größenverhältnis | 23,9 | 1,9 | 1,5 | 1,4 | 1,5 | 1,5 | 1,6 |
| PDI nach Präparation | 0,095 ± 0,007 | | | | | | |
| PDI nach Lyophilisierung | 0,669 ± 0,131 | 0,387 ± 0,050 | 0,272 ± 0,046 | 0,190 ± 0,065 | 0,217 ± 0,049 | 0,230 ± 0,064 | 0,240 ± 0,064 |
| PDI Verhältnis | 7,0 | 4,1 | 2,9 | 2 | 2,3 | 2,4 | 2,5 |
| Zeta Potential nach Präparation [mV] | -29,6 ± 0,2 | | | | | | |
| Zeta Potential nach Lyophilisierung [mV] | -29,7 ± 4,4 | -29,2 ± 2,5 | -30,9 ± 9,4 | -21,1 ± 6,7 | -19,7 ± 12,1 | -23,1 ± 10,7 | -23,9 ± 7,9 |
| Zeta Potential Verhältnis | 1,0 | 1,0 | 1,0 | 0,7 | 0,7 | 0,8 | 0,8 |

| | | | | | | | |
|---|---|---|---|---|---|---|---|
| ³⁾ PLGA = Milchsäure-Glycolsäure-Copolymer | | | | | | | |

### Beispiel 3a: Herstellung von Nanopartikelsuspensionen durch Nanofällung

5 mg PLGA³⁾ wurden in 2,5 mL Aceton gelöst und anschließend mittels Spritzenpumpe bei einer definierten Geschwindigkeit unter ständigem Rühren in ein Gefäß mit 4,5 mL einer wässrigen P(EtOx)₆₁ Lösung gegeben. Das organische Lösungsmittel wurde dann über Nacht unter ständigem Rühren abgedampft und die Nanopartikel wurden bezüglich ihres Partikeldurchmessers und des PDI charakterisiert.

**Tabelle 2: Ergebnisse der Herstellung von PLGA³⁾ Nanopartikeln mittels Nanofällung mit unterschiedlichen Konzentrationen P(EtOx)ₙ als Tensid.**

| **Konzentration Tensid [mg mL⁻¹]** | **0** | **0,3** | **0,5** | **1** |
|---|---|---|---|---|
| Größe [nm] | 98,0 ± 9,9 | 118,0 ± 0,7 | 122,2 ± 5,9 | 116,6 ± 5,2 |
| PDI | 0,103 ± 0,020 | 0,093 ± 0,007 | 0,153 ± 0,043 | 0,086 ± 0,017 |

| | | | | |
|---|---|---|---|---|
| ³⁾ PLGA = Milchsäure-Glycolsäure-Copolymer | | | | |

### Beispiel 3b: Herstellung von Nanopartikelsuspensionen durch Nanoemulsion

Eine definierte Menge P(EtOx)₆₁ wurde in 1 mL Reinstwasser gelöst. 10 mg PLGA³⁾ wurden in 0,5 mL Ethylacetat gelöst, vorsichtig auf die Tensidlösung pipettiert und anschließend mit dem Ultraschallfinger behandelt (Power: 40 W, Cycle: 100%, Amplitude: 100%, Time: 10 sec). Anschließend wurde die Partikelsuspension mit Reinstwasser um den Faktor 10 verdünnt und über Nacht bei Raumtemperatur gerührt, um das organische Lösungsmittel zu verdampfen und die Nanopartikel wurden bezüglich ihres Partikeldurchmessers und des PDI charakterisiert.

**Tabelle 3: Ergebnisse der Herstellung von PLGA³⁾ Nanopartikeln mittels Nanoemulsion mit unterschiedlichen Konzentrationen P(EtOx)₆₁ als Surfactant.**

| **Konzentration Tensid [%]** | **0** | **0,3** | **0,5** | **1** |
|---|---|---|---|---|
| Größe [nm] | 231,5 ± 15,3 | 448,9 ± 21,2 | 460,5 ± 48,5 | 217,4 ± 6,1 |
| PDI | 0,353 ± 0,010 | 0,383 ± 0,039 | 0,463 ± 0,061 | 0,164 ± 0,015 |

| | | | | |
|---|---|---|---|---|
| ³⁾ PLGA = Milchsäure-Glycolsäure-Copolymer | | | | |

### Beispiel 4a: Einfluss des DP von Poly(2-oxazolin) auf die Größe der Polymer-Nanopartikel nach Präparation mittels Nanofällung

5 mg PLGA³⁾ wurden in 2,5 mL Aceton gelöst und anschließend mittels Spritzenpumpe bei einer definierten Geschwindigkeit unter ständigem Rühren in ein Gefäß mit 4,5 mL einer wässrigen P(EtOx)ₙ oder P(MeOx)ₙ Lösung gegeben. Das organische Lösungsmittel wurde dann über Nacht unter ständigem Rühren abgedampft und die Nanopartikel wurden bezüglich ihres Partikeldurchmessers und des PDI mittels dynamischer Lichtstreuung charakterisiert. Ein Teil der Suspension wurde im -80 °C Gefrierschrank gefroren und anschließen über Nacht lyophilisiert. Ca. 2 mg des resultierenden Pulvers wurden dann in 1 mL Reinstwasser resuspendiert und erneut charakterisiert. Das Verhältnis der Werte wurde gebildet, indem die entsprechenden Messwerte nach der Lyophilisierung durch jene nach der Präparation dividiert wurden.

**Tabelle 4: Ergebnisse der Herstellung von PLGA³⁾ Nanopartikeln mittels Nanofällung mit P(EtOx)ₙ als Tensid in einer Konzentration von 1% (w/v).**

| **DP P(EtOx)ₙ** | **25** | **61** | **107** | **184** |
|---|---|---|---|---|
| Größe nach Präparation [nm] | 157,5 ± 7,2 | 118,0 ± 0,7 | 164,4 ± 11,5 | 172,5 ± 10,8 |
| Größe nach Lyophilisierung [nm] | 272,1 ± 167,8 | 134,5 ± 4,7 | 167,1 ± 15,7 | 173,4 ± 13,8 |
| Größenverhältnis | 1,7 | 1,4 | 1,02 | 1,0 |
| PDI nach Präparation | 0,071 ± 0,009 | 0,093 ± 0,007 | 0,076 ± 0,007 | 0,074 ± 0,010 |
| PDI nach Lyophilisierung | 0,261 ± 0,054 | 0,147 ± 0,052 | 0,114 ± 0,007 | 0,112 ± 0,002 |
| PDI Verhältnis | 3,7 | 1,6 | 1,5 | 1,5 |

**Tabelle 5: Ergebnisse der Herstellung von PLGA³⁾ Nanopartikeln mittels Nanofällung mit P(MeOx)ₙ als Tensid in einer Konzentration von 1% (w/v).**

| **DP P(MeOx)ₙ** | **25** | **57** | **100** | **211** |
|---|---|---|---|---|
| Größe nach Präparation [nm] | 169,8 ± 9,7 | 195,8 ± 6,0 | 172,9 ± 18,8 | 181,8 ± 5,9 |
| Größe nach Lyophilisierung [nm] | 299,4 ± 114,1 | 223,0 ± 36,6 | 181,6 ± 27,0 | 173,4 ± 15,9 |
| Größenverhältnis | 1,8 | 1,1 | 1,1 | 1,0 |
| PDI nach Präparation | 0,059 ± 0,006 | 0,069 ± 0,009 | 0,069 ± 0,015 | 0,124 ± 0,092 |
| PDI nach Lyophilisierung | 0,343 ± 0,069 | 0,125 ± 0,065 | 0,137 ± 0,023 | 0,108 ± 0,026 |
| PDI Verhältnis | 5,8 | 1,8 | 2,0 | 0,9 |

| | | | | |
|---|---|---|---|---|
| ³⁾ PLGA = Milchsäure-Glycolsäure-Copolymer | | | | |

### Beispiel 4b: Einfluss des DP von Poly(2-oxazolin) auf die Teilchengröße der Polymer-Nanoteilchen nach Präparation mittels Nanoemulsion

Eine definierte Menge P(EtOx)ₙ oder P(MeOx)ₙ wurde in 1 mL Reinstwasser gelöst. 10 mg PLGA³⁾ wurden in 0,5 mL Ethylacetat gelöst, vorsichtig auf die Surfactantlösung pipettiert und anschließend mit dem Ultraschallfinger behandelt (Leistung: 40 W, Zyklus: 100%, Amplitude: 100%, Zeit: 10 Sek). Anschließend wurde die Partikelsuspension mit Reinstwasser um den Faktor 10 verdünnt und über Nacht bei Raumtemperatur gerührt, um das organische Lösungsmittel zu verdampfen und die Nanopartikel wurden bezüglich ihres Partikeldurchmessers und des PDI charakterisiert. Ein Teil der Suspension wurde im -80 °C Gefrierschrank gefroren und anschließen über Nacht lyophilisiert. Ca. 2 mg der Partikel wurden dann in 1 mL Reinstwasser resuspendiert und erneut charakterisiert. Das Verhältnis der Werte wurde gebildet, indem die entsprechenden Messwerte nach der Lyophilisierung durch jene nach der Präparation dividiert wurden.

**Tabelle 6: Ergebnisse der Herstellung von PLGA³⁾ Nanopartikeln mittels Nanoemulsion mit P(EtOx)ₙ als Tensid in einer Konzentration von 1% (w/v). n.d.: nicht bestimmt (not determined).**

| **DP P(EtOx)ₙ** | **25** | **61** | **107** | **184** |
|---|---|---|---|---|
| Größe nach Präparation [nm] | 808,1 ± 362,7 | 217,4 ± 6,1 | 230,2 ± 18,5 | 194,2 ± 10,3 |
| Größe nach Lyophilisierung [nm] | n.d. | 204,4 ± 4,3 | 213,1 ± 12,8 | 179,2 ± 13,1 |
| Größenverhältnis | n.d. | 0,9 | 0,9 | 0,9 |
| PDI nach Präparation | 0,754 ± 0,426 | 0,164 ± 0,015 | 0,146 ± 0,047 | 0,086 ± 0,011 |
| PDI nach Lyophilisierung | n.d. | 0,100 ± 0,017 | 0,113 ± 0,010 | 0,102 ± 0,008 |
| PDI Verhältnis | n.d. | 0,6 | 0,8 | 1,2 |

**Tabelle 7: Ergebnisse der Herstellung von PLGA³⁾ Nanopartikeln mittels Nanoemulsion mit P(MeOx)ₙ als Tensid in einer Konzentration von 1% (w/v). n.d.: nicht bestimmt (not determined).**

| **DP P(MeOx)ₙ** | **25** | **57** | **100** | **211** |
|---|---|---|---|---|
| Größe nach Präparation [nm] | 803,7 ± 231,8 | 159,7 ± 4,0 | 273,2 ± 20,7 | 200,5 ± 5,3 |
| Größe nach Lyophilisierung [nm] | n.d. | 164,4 ± 6,7 | 269,9 ± 32,5 | 191,8 ± 7,2 |
| Größenverhältnis | n.d. | 1,0 | 1,0 | 1,0 |
| PDI nach Präparation | 0,289 | 0,086 ± 0,003 | 0,165 ± 0,007 | 0,080 ± 0,004 |
| PDI nach Lyophilisierung | n.d. | 0,108 ± 0,019 | 0,185 ± 0,083 | 0,100 ± 0,013 |
| PDI Verhältnis | n.d. | 1,3 | 1,1 | 1,3 |

| | | | | |
|---|---|---|---|---|
| ³⁾ PLGA = Milchsäure-Glycolsäure-Copolymer | | | | |

### Beispiel 5: Nanoteilchen aus unterschiedlichen Hüllpolymeren

100 mg P(EtOx)ₙ oder P(MeOx)ₙ wurden in 1 mL Reinstwasser gelöst. 10 mg Hüllpolymer wurden in 0.5 mL Ethylacetat gelöst, vorsichtig auf die Tensidlösung pipettiert und anschließend mit dem Ultraschallfinger behandelt (Leistung: 40 W, Zyklus: 100%, Amplitude: 100%, Zeit: 10 Sek). Anschließend wurde die Partikelsuspension mit Reinstwasser um den Faktor 10 verdünnt und über Nacht bei Raumtemperatur gerührt, um das organische Lösungsmittel zu verdampfen und die Nanopartikel wurden bezüglich ihres Partikeldurchmessers und des PDI charakterisiert. Die Nanopartikel wurden wie in der Tabelle beschrieben unterschiedlich aufgereinigt. Danach wurden alle resultieren Partikelsuspensionen im -80 °C Gefrierschrank gefroren und anschließen über Nacht lyophilisiert. Ca. 2 mg des Pulvers wurden dann in 1 mL Reinstwasser resuspendiert und erneut charakterisiert. Das Verhältnis der Werte wurde gebildet, indem die entsprechenden Messwerte nach der Lyophilisierung durch jene nach der Präparation dividiert wurden.

**Tabelle 8: Eigenschaften der Nanopartikel unterschiedlicher Hüllpolymere unter Nutzung von P(EtOx)₆₁ oder P(MeOx)₅₇ als Tensid nach der Präparation mittels Nanoemulsion. n.a.: nicht verfügbar (not available, da die Nanopartikel zu stark aggregierten).**

| **Hüllpolymer** | **Tensid** | **Größe [d, nm]** | **PDI** | **Zeta Potential [mV]** |
|---|---|---|---|---|
| PLGA³⁾ | P(EtOx)₆₁ | 214,9 ± 1,5 | 0,147 ± 0,030 | -33,0 ± 0,9 |
| | P(MeOx)₅₇ | 155,8 ± 0,9 | 0,087 ± 0,023 | -37,9 ± 0,5 |
| | Keins | n.a. | n.a. | n.a. |
| Eudragit^{®} RS 100⁴⁾ | P(EtOx)₆₁ | 214,0 ± 0,7 | 0,063 ± 0,018 | 35,7 ± 0,4 |
| | P(MeOx)₅₇ | 244,3 ± 2,5 | 0,081 ± 0,012 | 42,1 ± 1,7 |
| | Keins | 101,6 ± 0,5 | 0,246 ± 0,020 | 58,6 ± 0,4 |
| P(MMA₉₇-*co*-MAEMA₃₂)⁵⁾ | P(EtOx)₆₁ | 155,2 ± 2,8 | 0,165 ± 0,012 | 32,3 ± 0,1 |
| | P(MeOx)₅₇ | 162,9 ± 1,3 | 0,138 ± 0,028 | 33,6 ± 1,4 |
| | Keins | 176,2 ± 2,4 | 0,191 ± 0,012 | 49,1 ± 0,4 |

**Tabelle 9: Eigenschaften der Nanopartikel unterschiedlicher Hüllpolymere unter Nutzung von P(EtOx)₆₁ oder P(MeOx)₅₇ als Tensid nach der Aufreinigung und Lyophilisierung. P(MeOx)₅₇ ist ein Poly(2-methyl-2-oxazolin) mit einem Polymerisationsgrad (DP) von 57. n.a.: nicht verfügbar (not available, da die Nanopartikel zu stark aggregierten).**

| **P(MMA₉₇-*co*-MAEMA₃₂)⁵⁾** | | | | | | |
|---|---|---|---|---|---|---|
| **Tensid** | **P(EtOx)₆₁** | | **P(MeOx)₅₇** | | **Keins** | |
| *Aufreinigungsmethode* | *Größe [d, nm]* | *PDI* | *Größe [d, nm]* | *PDI* | *Größe [d, nm]* | *PDI* |
| Nach Präparation | 155,2 ± 2,8 | 0,165 ± 0,012 | 162,9 ± 1,3 | 0,138 ± 0,028 | 176,2 ± 2,4 | 0,191 ± 0,012 |
| Lyophilisierung ohne Aureinigung | 172,2 ± 2,7 | 0,215 ± 0,021 | 157,5 ± 1,9 | 0,109 ± 0,026 | n.a. | n.a. |
| Zentrifugation und Resuspension in 1 mL Reinstwasser | 229,2 ± 14,9 | 0,330 ± 0,019 | 294,7 ± 67,1 | 0,375 ± 0,033 | 325,8 ± 88,1 | 0,362 ± 0,034 |
| Zentrifugation und Resuspension in 1 mL 0,5% POx Lösung | 175,4 ± 2,3 | 0,258 ± 0,035 | 179,7 ± 3,8 | 0,128 ± 0,083 | 253,4 ± 4,1* | 0,278 ± 0,042* |
| Spritzenfiltration | n.a. | n.a. | 211,6 ± 3,1 | 0,269 ± 0,032 | n.a. | n.a. |

| **Eudragit^{®} RS100⁴⁾** | | | | | | |
|---|---|---|---|---|---|---|
| **Tensid** | **P(EtOx)₆₁** | | **P(MeOx)₅₇** | | **Keins** | |
| *Aufreinigungsmethode* | *Größe [d, nm]* | *PDI* | *Größe [d, nm]* | *PDI* | *Größe [d, nm]* | *PDI* |
| Nach Präparation | *214,0* ± 0,7 | *0,063* ± 0,018 | *244,3* ± 2,5 | *0,081* ± 0,012 | *101,6* ± 0,5 | *0,246 ±̅* 0,020 |
| Lyophilisierung ohne Aureinigung | 217,2 ± 2,2 | 0,080 ± 0,024 | *247,3* ± 4,3 | 0,086 ± 0,038 | n.a. | n.a. |
| Zentrifugation und Resuspension in 1 mL Reinstwasser | 239,3 ± 1,7 | 0,105 ± 0,037 | 315,9 ± 8,3 | 0,220 ± 0,044 | n.a. | n.a. |
| Zentrifugation und Resuspension in 1 mL 0,5% POx Lösung | 223,3 ± 1,5 | 0,069 ± 0,026 | 252,0 ± 3,4 | 0,105 ± 0,039 | n.a. | n.a. |
| Spritzenfiltration | 220,4 ± 4,4 | 0,062 ± 0,042 | 257,5 ± 3,7 | 0,068 ± 0,033 | n.a. | n.a. |

**Tabelle 10: Verhältnisse der Eigenschaften der Nanopartikel unterschiedlicher Hüllpolymere unter Nutzung von P(EtOx)₆₁ oder P(MeOx)₅₇ als Tensid nach der Aufreinigung und Lyophilisierung. P(MeOx)₅₇ ist ein Poly(2-methyl-2-oxazolin) mit einem Polymerisationsgrad (DP) von 57. n.a.: nicht verfügbar (not available, da die Nanopartikel zu stark aggregierten).**

| **P(MMA₉₇-*co*-MAEMA₃₂)⁵⁾** | | | | | | |
|---|---|---|---|---|---|---|
| **Tensid** | **P(EtOx)₆₁** | | **P(MeOx)₅₇** | | **Keins** | |
| *Aufreinigungsmethode* | *Größenverhältnis* | *PDI Verhältnis* | *Größenverhältnis* | *PDI Verhältnis* | *Größenverhältnis* | *PDI Verhältnis* |
| Lyophilisierung ohne Aufreinigung | 1,11 | 1,30 | 0,97 | 0,79 | n.a. | n.a. |
| Zentrifugation und Resuspension in 1 mL Reinstwasser | 1,48 | 2,00 | 1,01 | 1,06 | 1.85 | 1.90 |
| Zentrifugation und Resuspension in 1 mL 0,5% POx Lösung | 1,13 | 1,56 | 1,10 | 0,93 | 1,44* | 1,46* |
| Spritzenfiltration | n.a. | n.a. | 1,30 | 1,95 | n.a. | n.a. |

| **Eudragit^{®} RS100⁴⁾** | | | | | | |
|---|---|---|---|---|---|---|
| **Tensid** | **P(EtOx)₆₁** | | **P(MeOx)₅₇** | | **Keins** | |
| *Aufreinigungsmethode* | *Größenverhältnis* | *PDI Verhältnis* | *Größenverhältnis* | *PDI Verhältnis* | *Größenverhältnis* | *PDI Verhältnis* |
| Lyophilisierung ohne Aufreinigung | 1,01 | 1,27 | 1,01 | 1,06 | n.a. | n.a. |
| Zentrifugation und Resuspension in 1 mL Reinstwasser | 1,12 | 1,67 | 1,29 | 2,71 | n.a. | n.a. |
| Zentrifugation und Resuspension in 1 mL 0,5% POx Lösung | 1,04 | 1,10 | 1,03 | 1,30 | n.a. | n.a. |
| Spritzenfiltration | 1,03 | 0,98 | 1,05 | 0,84 | n.a. | n.a. |

| | | | | | | |
|---|---|---|---|---|---|---|
| ³⁾PLGA = Milchsäure-Glycolsäure-Copolymer ⁴⁾ Eudragit^{®} RS 100 = Methylmethacrylat-(2-(N,N,N-trimethylammoniumethyl))methacrylat-ethylacrylat-Copolymer ⁵⁾ P(MMA₉₇-*co*-MAEMA₃₂) = 2-(N-Methylaminethyl)methacrylat-methylacrylat-Copolymer | | | | | | |

### Beispiel 6: Einfluss der Aufreinigung auf die Größe der Nanoteilchen

5 mg PLGA³⁾ wurden in 2,5 mL Aceton gelöst und anschließend mittels Spritzenpumpe bei einer definierten Geschwindigkeit unter ständigem Rühren in ein Gefäß mit 4,5 mL einer wässrigen P(EtOx)₆₁ Lösung gegeben. Das organische Lösungsmittel wurde dann über Nacht unter ständigem Rühren abgedampft und die Nanopartikel wurden bezüglich ihres Partikeldurchmessers und des PDI charakterisiert. Die Nanopartikel wurden wie in der Tabelle beschrieben unterschiedlich aufgereinigt. Danach wurden alle resultieren Partikelsuspensionen im -80 °C Gefrierschrank gefroren und anschließen über Nacht lyophilisiert. Ca. 2 mg des Pulvers wurden dann in 1 mL Reinstwasser resuspendiert und erneut charakterisiert. Das Verhältnis der Werte wurde gebildet, indem die entsprechenden Messwerte nach der Lyophilisierung durch jene nach der Präparation dividiert wurden.

**Tabelle 11: Eigenschaften der PLGA³⁾ Nanopartikel unter Nutzung von P(EtOx)₆₁ oder P(MeOx)₅₇ als Tensid nach der Aufreinigung und Lyophilisierung.**

| **Tensid** | **P(EtOx)₆₁** | | **P(MeOx)₅₇** | |
|---|---|---|---|---|
| *Aufreinigungsmethode vor Lyophilisierung* | *Größe [d, nm]* | *PDI* | *Größe [d, nm]* | *PDI* |
| Nach Präparation | 157,7 ± 1,3 | 0,102 ± 0,017 | 201,7 ± 4,2 | 0,062 ± 0,029 |
| Lyophilisierung ohne Aufreinigung | 1262 ± 1182 | 0,714 ± 0,201 | 213,5 ± 3,1 | 0,067 ± 0,010 |
| Zentrifugation und Resuspension in 1 mL Reinstwasser | 898 ± 1062 | 0,549 ± 0,305 | 206,7 ± 3,1 | 0,111 ± 0,034 |
| Spritzenfiltration und Zugabe von 1 mL 0,5% POx Lösung | 165,1 ± 1,0 | 0,172 ± 0,044 | 189,6 ± 1,2 | 0,068 ± 0,018 |
| Spritzenfiltration | 1075 ± 111,6 | 0,948 ± 0,100 | 190,6 ± 1,5 | 0,101 ± 0,019 |

**Tabelle 12: Verhältnisse der Eigenschaften der PLGA³⁾ Nanopartikel unter Nutzung von P(EtOx)₆₁ oder P(MeOx)₅₇ als Tensid nach der Aufreinigung und Lyophilisierung. n.a.: nicht verfügbar (not available, da die Nanopartikel zu stark aggregierten).**

| **Additive** | **P(EtOx)₆₁** | | **P(MeOx)₅₇** | |
|---|---|---|---|---|
| *Aufreinigungsmethode* | *Größenverhältnis* | *PDI Verhältnis* | *Größenverhältnis* | *PDI Verhältnis* |
| Lyophilisierung ohne Aufreinigung | 8,00 | 7,00 | 1,06 | 1,08 |
| Zentrifugation und Resuspension in 1 mL Reinstwasser | 5,69 | 5,38 | 1,02 | 1,79 |
| Spritzenfiltration und Zugabe von 1 mL 0,5% POx Lösung | 1,05 | 1,69 | 0,94 | 1,10 |
| Spritzenfiltration | 6,82 | 9,29 | 0,94 | 1,63 |

| | | | | |
|---|---|---|---|---|
| ³⁾ PLGA = Milchsäure-Glycolsäure-Copolymer | | | | |

### Beispiel 7: Einkapselung von Nilrot

Aus 10 mg PLGA³⁾ und 0,1 mg Nilrot aus einer 1 mg mL⁻¹ Vorratslösung in Aceton wurden wie in Beispiel 3 Nanopartikel mittels Nanofällung oder Nanoemulsion hergestellt. Als Tensidkonzentration (sofern in der wässrigen Lösung vorhanden) wurde eine finale Konzentration von 1% für die Nanoemulsion und 0,3% für die Nanofällung angestrebt.

Die Nanopartikel wurden wie in der Tabelle beschrieben unterschiedlich aufgereinigt. Danach wurden alle resultieren Partikelsuspensionen im -80 °C Gefrierschrank gefroren und anschließen über Nacht lyophilisiert. Ca. 2 mg des Pulvers wurden dann in 1 mL Reinstwasser resuspendiert und erneut charakterisiert. Das Verhältnis der Werte wurde gebildet, indem die entsprechenden Messwerte nach der Lyophilisierung durch jene nach der Präparation dividiert wurden. Ein Teil der resultierenden Nanopartikel wurde in DMF⁶⁾ gelöst und die Einkapselungseffizienz des Wirkstoffes mittels UV/VIS-Spektroskopie über dessen Absorption bestimmt.

**Tabelle 13: Eigenschaften der PLGA³⁾-Nilrot Nanopartikel unter Nutzung von P(EtOx)₆₁ oder P(MeOx)₅₇ als Tensid nach der Präparation mittels Nanoemulsion und Nanofällung. n.a. = nicht verfügbar (not available).**

| **Wirkstoff** | **Tensid** | **Präparationsmethode** | **Größe [d, nm]** | **PDI** |
|---|---|---|---|---|
| Nilrot | P(EtOx)₆₁ | Nanofällung | 160,8 ± 1,5 | 0,053 ± 0,028 |
| Nilrot | P(EtOx)₆₁ | Nanoemulsion | 190,7 ± 1,9 | 0,124 ± 0,013 |
| Nilrot | P(MeOx)₅₇ | Nanofällung | 151,2 ± 0,8 | 0,065 ± 0,021 |
| Nilrot | P(MeOx)₅₇ | Nanoemulsion | 180,0 ± 0,8 | 0,099 ± 0,014 |
| Nilrot | Keins | Nanofällung | 145,2 ± 2,5 | 0,075 ± 0,018 |
| Nilrot | Keins | Nanoemulsion | n.a. | n.a. |

**Tabelle 14: Eigenschaften der PLGA³⁾-Nilrot Nanopartikel unter Nutzung von P(EtOx)₆₁ oder P(MeOx)₅₇ als Tensid nach der Aufreinigung und Lyophilisierung. n.a.: nicht verfügbar (not available, da die Nanopartikel zu stark aggregierten). n.r.: nicht resuspendierbar.**

| **Additiv** | **P(EtOx)₆₁** | | **P(MeOx)₅₇** | | **Keins** | |
|---|---|---|---|---|---|---|
| *Aufreinigungsmethode* | *Größe [d, nm]* | *PDI* | *Größe [d, nm]* | *PDI* | *Größe [d, nm]* | *PDI* |
| Nach der Präparation | 160,8 ± 1,5 | 0,053 ± 0,028 | 169,0 ± 2,5 | 0,069 ± 0,021 | 145,2 ± 2,5 | 0,075 ± 0,018 |
| Lyophilisierung ohne Aureinigung | 168,0 ± 1,6 | 0,087 ± 0,031 | n.a. | n.a. | n.a. | n.a. |
| Zentrifugation und Resuspension in 1 mL 0,5% POₓ Lösung | 184,3 ± 1,6 | 0,145 ± 0,028 | 187,4 ± 3,2 | 0,123 ± 0,052 | n.r. | n.r. |
| Spritzenfiltration und Zugabe von 1 mL 0.5% POₓ Lösung | 167,7 ± 3,2 | 0,148 ± 0,045 | 233,1 ± 8,4 | 0,314 ± 0,022 | 180,5 ± 1,6 | 0,169 ± 0,020 |
| Spritzenfiltration | 161,7 ± 3,4 | 0,157 ± 0,011 | n.a. | n.a. | n.a. | n.a. |

**Tabelle 15: Verhältnisse der Eigenschaften der PLGA³⁾-Nilrot Nanopartikel unter Nutzung von P(EtOx)₆₁ oder P(MeOx)₅₇ als Tensid nach der Aufreinigung und Lyophilisierung. EE: Einkapselungseffizienz.**

| **Additiv** | **P(EtOx)₆₁** | | | **P(MeOx)₅₇** | | | **Keins** | | |
|---|---|---|---|---|---|---|---|---|---|
| *Aufreinigungsmethode* | *Größen verhältnis* | *PDI Verhältnis* | *EE [µg mg⁻¹]* | *Größenverhältnis* | *PDI Verhältnis* | *EE [µg mg⁻¹]* | *Größenverhältnis* | *PDI Verhältnis* | *EE [µg mg⁻¹]* |
| Lyophilisierung ohne Aufreinigung | 1,04 | 1,64 | 0,52 | n.a. | n.a. | n.a. | 28,21 | 12,23 | 1,53 |
| Zentrifugation und Resuspension in 1 mL 0,5% POx Lösung | 1,14 | 2,74 | 0,28 | 1,15 | 1,71 | 0,32 | n.a. | n.a. | n.a. |
| Spritzenfiltration und Zugabe von 1 mL 0,5% POx Lösung | 1,04 | 2,79 | 0,21 | n.a. | n.a. | n.a. | 1,24 | 2,25 | 0,28 |
| Spritzenfiltration | 1,01 | 2,96 | 0,51 | n.a. | n.a. | n.a. | n.a. | n.a. | n.a. |

| | | | | | | | | | |
|---|---|---|---|---|---|---|---|---|---|
| ³⁾ PLGA = Milchsäure-Glycolsäure-Copolymer ⁶⁾ DMF = Dimethylformamid | | | | | | | | | |

### Beispiel 8: Einkapselung von PKC 412⁷⁾

10 mg PLGA³⁾ wurden in 1 mL Aceton gelöst. 0,3 mg PKC 412⁷⁾ wurden in 30 µL DMSO⁸⁾ gelöst und zu der PLGA³⁾ Lösung gegeben. Dann wurde die Polymer-Wirkstoff Lösung in mittels Spritzenpumpe bei einer definierten Geschwindigkeit unter ständigem Rühren in ein Gefäß mit 10 mL einer wässrigen Lösung, die gegebenenfalls ein Tensid enthielt, gegeben. Das organische Lösungsmittel wurde dann über Nacht unter ständigem Rühren abgedampft und die Nanopartikel wurden bezüglich ihres Partikeldurchmessers und des PDI charakterisiert. Danach wurden alle resultieren Partikelsuspensionen im -80 °C Gefrierschrank gefroren und anschließen über Nacht lyophilisiert. Ca. 2 mg der Partikel wurden dann in 1 mL Reinstwasser oder einer 0,5%igen Lösung des entsprechenden Poly(2-oxazolin)s resuspendiert und erneut charakterisiert.

Das Verhältnis der Werte wurde gebildet, indem die entsprechenden Messwerte nach der Lyophilisierung durch jene nach der Präparation dividiert wurden. Ein Teil der resultierenden Nanopartikel wurde in DMSO⁸⁾ gelöst und die Einkapselungseffizienz des Wirkstoffes mittels UV/VIS-Spektroskopie über dessen Absorption bestimmt.

**Tabelle 16: Eigenschaften der PLGA-PKC 412^{3,7)} Nanopartikel unter Nutzung von P(EtOx)₆₁ oder P(MeOx)₅₇ als Tensid nach der Aufreinigung und Lyophilisierung. n.a.: nicht verfügbar (not available, da die Nanopartikel zu stark aggregierten).EE: Einkapselungseffizienz.**

| **Tensid** | **Nach Präparation** | | **Nach Lyophilisierung** | | **Verhältnis** | | |
|---|---|---|---|---|---|---|---|
| | *Größe [d, nm]* | *PDI* | *Größe [d, nm]* | *PDI* | *Größe* | *PDI* | *EE [µg mg⁻¹]* |
| **P(EtOx)₆₁** | 168,6 ± 2,4 | 0,061 ± 0,019 | 178,9 ± 2,0 | 0,058 ± 0,025 | 1,06 | 0,95 | 0 |
| **P(MeOx)₅₇** | 184,6 ± 1,1 | 0,063 ± 0,034 | 190,4 ± 2,4 | 0,086 ± 0,022 | 1,03 | 1,36 | 0,92 ± 0,08 |
| **Keins** | 156,1 ± 0,9 | 0,077 ± 0,024 | 7272 ± 2494 | 0,740 ± 0,383 | n.a. | n.a. | 0,98 ± 0,31 |

| | | | | | | | |
|---|---|---|---|---|---|---|---|
| ³⁾ PLGA = Milchsäure-Glycolsäure-Copolymer ⁷⁾ PKC 412 = [9*S*-(9α,10β,11β,13α)]-*N*-(2,3,10,11,12,13-Hexahydro-10-methoxy-9-methyl-1-oxo-9,13-epoxy-1*H*,9*H*-diindolo[1,2,3-*gh*:3',2',1'-*lm*]pyrrolo[3,4-*j*][1,7]benzodiazonin-11-yl)-*N*-methylbenzamid ⁸⁾ DMSO = Dimethylsulfoxid | | | | | | | |

## Patentansprüche

1. Zusammensetzungen enthaltend wasserlösliches Poly(oxazolin) und organische Polymerpartikel ausgewählt aus der Gruppe der von organischen Dicarbonsäuren und organischen Diolen und/oder von organischen Hydroxycarbonsäuren abgeleiteten Polyester, der Polycarbonate, der von Estern ethylenisch ungesättigter Carbonsäuren abgeleiteten Polymere oder aus einem Gemisch von zwei oder mehreren dieser Polymere.

2. Zusammensetzungen nach Anspruch 1, **dadurch gekennzeichnet, dass** die Polymerpartikel ausgewählt werden aus der Gruppe der von aliphatischen, cycloaliphatischen und/oder aromatischen Dicarbonsäuren oder deren polyesterbildenden Derivaten und aliphatischen oder cycloaliphatischen Diolen und/oder von aliphatischen oder cycloaliphatischen Hydroxymonocarbonsäuren oder deren polyesterbildenden Derivaten abgeleiteten Polyester oder der von Estern ethylenisch ungesättigter Carbonsäuren abgeleitete Polymere.

3. Zusammensetzungen nach mindestens einem der Ansprüche 1 oder 2, **dadurch gekennzeichnet, dass** der mittlere Durchmesser D₅₀ der organischen Polymerpartikel weniger als 10 µm beträgt, vorzugsweise weniger als 1 µm.

4. Zusammensetzungen nach mindestens einem der Ansprüche 1 bis 3, **dadurch gekennzeichnet, dass** die organischen Polymerpartikel Durchmesser im Bereich von 50 bis 999 nm aufweisen.

5. Zusammensetzungen nach mindestens einem der Ansprüche 1 bis 4, **dadurch gekennzeichnet, dass** die organischen Polymerpartikel eine disperse Phase in einer hydrophilen Flüssigkeit bilden.

6. Zusammensetzungen nach Anspruch 5, **dadurch gekennzeichnet, dass** die organischen Polymerpartikel in der hydrophilen Flüssigkeit suspendiert sind.

7. Zusammensetzungen nach mindestens einem der Ansprüche 1 bis 6, **dadurch gekennzeichnet, dass** die organischen Polymerpartikel in fester Form vorliegen und von dem Poly(oxazolin) umhüllt sind.

8. Zusammensetzungen nach mindestens einem der Ansprüche 1 bis 7, **dadurch gekennzeichnet, dass** das organische Polymerpartikel einen von organischen Dicarbonsäuren und organischen Diolen und/oder von organischen Hydroxycarbonsäuren abgeleiteten Polyester enthält.

9. Zusammensetzungen nach Anspruch 8, **dadurch gekennzeichnet, dass** der Polyester ein Polyhydroxyalkanoat ist, insbesondere ein Milchsäurehomo- oder - copolymer.

10. Zusammensetzungen nach mindestens einem der Ansprüche 1 bis 7, **dadurch gekennzeichnet, dass** das organische Polymerpartikel ein von Estern ethylenisch ungesättigter Carbonsäuren abgeleitetes Polymer enthält, insbesondere ein von Acrylsäureestern und/oder von Methacrylsäureestern abgeleitetes Homo- oder Copolymer, ganz besonders bevorzugt ein Acrylsäurealkylester-Methylacrylsäurealkylester-Copolymer.

11. Zusammensetzungen nach mindestens einem der Ansprüche 1 bis 10, **dadurch gekennzeichnet, dass** das wasserlösliche Poly(oxazolin) mindestens 80 Gew.-%, bezogen auf dessen Gesamtmasse, an wiederkehrenden Struktureinheiten der Formel I und/oder der Formel II aufweist
-NR¹-CR³H-CR⁴H- (I),
-NR¹-CR³H-CR⁴H-CR⁵H- (II),
worin
R¹ einen Rest der Formel -CO-R² bedeutet,
R³, R⁴ und R⁵ unabhängig voneinander Wasserstoff, Methyl, Ethyl, Propyl oder Butyl bedeuten,
R² ausgewählt wird aus der Gruppe bestehend aus Wasserstoff, Methyl, Ethyl,
-CₘH₂ₘ-X oder -(CₙH₂ₙ-O)ₒ-(CₚH₂ₚ-O)_{q}-R⁶,
R⁶ Wasserstoff oder C₁-C₆-Alkyl ist,
m eine ganze Zahl von 1 bis 6 ist,
X ausgewählt wird aus der Gruppe bestehend aus Hydroxyl, Alkoxy, Amino, N-Alkylamino, N,N-Dialkylamino, Carboxyl, Carbonsäureester, Sulfonyl, Sulfonsäureester oder Carbamat,
n und p unabhängig voneinander ganze Zahlen von 2 bis 4 sind, wobei n ungleich p ist, und
o und q unabhängig ganze Zahlen von 0 bis 60 sind, wobei mindestens eines der o oder q ungleich 0 ist.

12. Zusammensetzungen nach Anspruch 11, **dadurch gekennzeichnet, dass** R² Wasserstoff, Methyl oder Ethyl ist und R³ bis R⁵ Wasserstoff bedeuten oder in denen R² Wasserstoff, Methyl oder Ethyl ist und zwei der Reste R³ bis R⁵ Wasserstoff sind ist und einer der Reste R³ bis R⁵ Methyl oder Ethyl ist.

13. Zusammensetzungen nach mindestens einem der Ansprüche 11 oder 12, **dadurch gekennzeichnet, dass** das wasserlösliche Poly(oxazolin) mindestens 90 Gew.-%, bezogen auf dessen Gesamtmasse, an wiederkehrenden Struktureinheiten der Formel I aufweist, worin R² Methyl oder Ethyl bedeutet.

14. Zusammensetzungen nach mindestens einem der Ansprüche 1 bis 13, **dadurch gekennzeichnet, dass** die organischen Polymerpartikel einen oder mehrere pharmazeutische Wirkstoffe enthalten.

15. Verwendung von wasserlöslichen Poly(oxazolin)en zur Stabilisierung von organischen Polymerpartikeln ausgewählt aus der Gruppe der von organischen Dicarbonsäuren und organischen Diolen und/oder von organischen Hydroxycarbonsäuren abgeleiteten Polyester, der Polycarbonate, der von Estern ethylenisch ungesättigter Carbonsäuren abgeleiteten Polymere, oder aus einem Gemisch von zwei oder mehreren dieser Polymere in einer hydrophilen Flüssigkeit.

16. Verwendung von wasserlöslichen Poly(oxazolin)en zur Stabilisierung von organischen Polymerpartikeln ausgewählt aus der Gruppe der von organischen Dicarbonsäuren und organischen Diolen und/oder von organischen Hydroxycarbonsäuren abgeleiteten Polyester, der Polycarbonate, der von Estern ethylenisch ungesättigter Carbonsäuren abgeleiteten Polymere oder aus einem Gemisch von zwei oder mehreren dieser Polymere bei der Gefriertrocknung .

17. Verwendung von wasserlöslichen Poly(oxazolin)en als Stabilisatoren bei der Herstellung oder bei der Aufarbeitung von organischen Polymerpartikeln ausgewählt aus der Gruppe der von organischen Dicarbonsäuren und organischen Diolen und/oder von organischen Hydroxycarbonsäuren abgeleiteten Polyester, der Polycarbonate, der von Estern ethylenisch ungesättigter Carbonsäuren abgeleiteten Polymere oder aus einem Gemisch von zwei oder mehreren dieser Polymere in einer hydrophilen Flüssigkeit.

18. Verwendung von wasserlöslichen Poly(oxazolin)en zur Stabilisierung von pulverförmigen organischen Polymerpartikeln ausgewählt aus der Gruppe der von organischen Dicarbonsäuren und organischen Diolen und/oder von organischen Hydroxycarbonsäuren abgeleiteten Polyester, der Polycarbonate, der von Estern ethylenisch ungesättigter Carbonsäuren abgeleiteten Polymere oder aus einem Gemisch von zwei oder mehreren dieser Polymere.

## Claims

1. Compositions comprising water-soluble poly(oxazoline) and organic polymer particles selected from the group of polyesters derived from organic dicarboxylic acids and organic diols and/or from organic hydroxycarboxylic acids, polycarbonates, polymers derived from esters of ethylenically unsaturated carboxylic acids or of a mixture of two or more of these polymers.

2. Compositions according to claim 1, whrein the polymer particles are selected from the group consisting of polyesters derived from aliphatic, cycloaliphatic and/or aromatic dicarboxylic acids or their polyester-forming derivatives and aliphatic or cycloaliphatic diols and/or from aliphatic or cycloaliphatic hydroxymonocarboxylic acids or their polyester-forming derivatives or from polymers derived from esters of ethylenically unsaturated carboxylic acids.

3. Compositions according to at least one of claims 1 or 2, wherein the mean diameter D₅₀ of the organic polymer particles is less than 10 µm, preferably less than 1 µm.

4. Compositions according to at least one of claims 1 to 3, wherein the organic polymer particles have diameters in the range between 50 and 999 nm.

5. Compositions according to at least one of claims 1 to 4, wherein the organic polymer particles form a dispersed phase in a hydrophilic liquid.

6. Compositions according to claim 5, wherein the organic polymer particles are suspended in the hydrophilic liquid.

7. Compositions according to at least one of claims 1 to 6, wherein the organic polymer particles are in solid form and are coated by the poly(oxazoline).

8. Compositions according to at least one of claims 1 to 7, wherein the organic polymer particle contains a polyester derived from organic dicarboxylic acids and organic diols and/or from organic hydroxycarboxylic acids.

9. Compositions according to claim 8, wherein the polyester is a polyhydroxy-alkanoate, in particular a lactic acid homopolymer or copolymer.

10. Compositions according to at least one of claims 1 to 7, wherein the organic polymer particle comprises a polymer derived from esters of ethylenically unsaturated carboxylic acids, in particular a homopolymer or copolymer derived from acrylic acid esters and/or methacrylic acid esters, most preferably an alkyl acrylate-alkyl methacrylate copolymer.

11. Compositions according to at least one of claims 1 to 10, wherein the water-soluble poly(oxazoline) comprises at least 80% by weight, based on its total mass, of recurring structural units of formula I and/or formula II
-NR¹-CR³H-CR⁴H- (I),
-NR¹-CR³H-CR⁴H-CR⁵H- (II),
wherein
R¹ represents a residue of the formula -CO-R²,
R³, R⁴ and R⁵ independently represent hydrogen, methyl, ethyl, propyl, or butyl, R² is selected from the group consisting of hydrogen, methyl, ethyl, -CₘH₂ₘ-X or
-(CₙH₂ₙ-O)ₒ-(CₚH₂ₚ-O)_{q}-R⁶,
R⁶ is hydrogen or C₁-C₆ alkyl,
m is an integer from 1 to 6,
X is selected from the group consisting of hydroxyl, alkoxy, amino, N-alkylamino, N,N-dialkylamino, carboxyl, carboxylic ester, sulfonyl, sulfonic ester, or carbamate,
n and p are independently integers from 2 to 4, where n is not equal to p, and
o and q are independently integers from 0 to 60, where at least one of o or q is not equal to 0.

12. Compositions according to claim 11, wherein R² is hydrogen, methyl, or ethyl, and R³ to R⁵ are hydrogen, or in which R² is hydrogen, methyl, or ethyl, and two of the residues R³ to R⁵ are hydrogen, and one of the residues R³ to R⁵ is methyl or ethyl.

13. Compositions according to at least one of claims 11 or 12, wherein the water-soluble poly(oxazoline) comprises at least 90% by weight, based on its total mass, of repeating structural units of formula I, wherein R² is methyl or ethyl.

14. Compositions according to at least one of claims 1 to 13, wherein the organic polymer particles comprise one or more pharmaceutical active ingredients.

15. Use of water-soluble poly(oxazoline)s for stabilizing organic polymer particles selected from the group of polyesters derived from organic dicarboxylic acids and organic diols and/or from organic hydroxycarboxylic acids, polycarbonates, polymers derived from esters of ethylenically unsaturated carboxylic acids, or of a mixture of two or more of these polymers in a hydrophilic liquid.

16. Use of water-soluble poly(oxazoline)s for stabilizing organic polymer particles selected from the group of polyesters derived from organic dicarboxylic acids and organic diols and/or from organic hydroxycarboxylic acids, polycarbonates, polymers derived from esters of ethylenically unsaturated carboxylic acids, or of a mixture of two or more of these polymers in freeze-drying.

17. Use of water-soluble poly(oxazoline)s as stabilizers in the manufacture or processing of organic polymer particles selected from the group of polyesters derived from organic dicarboxylic acids and organic diols and/or from organic hydroxycarboxylic acids, polycarbonates, polymers derived from esters of ethylenically unsaturated carboxylic acids, or of a mixture of two or more of these polymers in a hydrophilic liquid.

18. Use of water-soluble poly(oxazoline)s for stabilizing powdered organic polymer particles selected from the group of polyesters derived from organic dicarboxylic acids and organic diols and/or from organic hydroxycarboxylic acids, polycarbonates, polymers derived from esters of ethylenically unsaturated carboxylic acids, or of a mixture of two or more of these polymers.

## Revendications

1. Compositions contenant du poly(oxazoline) hydrosoluble et des particules de polymère organique sélectionnées parmi le groupe des polyesters dérivés d'acides dicarboxyliques organiques et de diols organiques et/ou d'acides hydroxycarboxyliques organiques, des polycarbonates, des polymères dérivés d'esters d'acides carboxyliques éthylène insaturés ou d'un mélange de deux ou plusieurs de ces polymères.

2. Compositions selon la revendication 1, **caractérisées en ce que** les particules de polymère sont choisies parmi le groupe constitué de polyesters dérivés d'acides dicarboxyliques aliphatiques, cycloaliphatiques et/ou aromatiques ou de leurs dérivés formant du polyester et de diols aliphatiques ou cycloaliphatiques et/ou d'acides hydroxymonocarboxyliques aliphatiques ou cycloaliphatiques ou de leurs dérivés formant du polyester ou de polymères dérivés d'esters d'acides carboxyliques insaturés d'éthylène.

3. Compositions selon au moins une des revendications 1 ou 2, **caractérisées en ce que** le diamètre moyen D₅₀ des particules de polymère organique est inférieur à 10 µm, de préférence inférieur à 1 µm.

4. Compositions selon au moins une des revendications 1 à 3, **caractérisées en ce que** les particules de polymère organique ont un diamètre compris entre 50 et 999 nm.

5. Compositions selon au moins une des revendications 1 à 4, **caractérisées en ce que** les particules de polymère organique forment une phase dispersée dans un liquide hydrophile.

6. Compositions selon la revendication 5, **caractérisées en ce que** les particules de polymère organique sont en suspension dans le liquide hydrophile.

7. Compositions selon au moins une des revendications 1 à 6, **caractérisées en ce que** les particules de polymère organique sont à l'état solide et sont enrobées de poly(oxazoline).

8. Compositions selon au moins une des revendications 1 à 7, **caractérisées en ce que** la particule de polymère organique contient un polyester dérivé d'acides dicarboxyliques organiques et de diols organiques et/ou d'acides hydroxycarboxyliques organiques.

9. Compositions selon la revendication 8, **caractérisées en ce que** le polyester est un polyhydroxyalcanoate, notamment un homopolymère ou un copolymère d'acide lactique.

10. Compositions selon au moins une des revendications 1 à 7, **caractérisées en ce que** la particule de polymère organique contient un polymère dérivé d'esters d'acides éthylènecarboxyliques insaturés, notamment un homopolymère ou un copolymère dérivé d'esters acryliques et/ou d'esters méthacryliques, de préférence un copolymère d'ester alkylique d'acide acrylique - ester alkylique d'acide méthacrylique

11. Compositions selon au moins une des revendications 1 à 10, **caractérisées en ce que** le poly(oxazoline) hydrosoluble comprend au moins 80 % en poids, par rapport à sa masse totale, d'unités structurales récurrentes de formule I et/ou de formule II.
-NR¹-CR³H-CR⁴H- (I),
-NR¹-CR³H-CR⁴H-CR⁵H- (II),
où
R¹ représente un résidu de formule -CO-R²,
R³, R⁴ et R⁵ représentent respectivement hydrogène, méthyle, éthyle, propyle ou butyle,
R² est choisi parmi l'hydrogène, le groupe méthyle, éthyle, -CₘH₂ₘ-X ou
-(CₙH₂ₙ-O)ₒ-(CₚH₂ₚ-O)_{q}-R⁶,
R⁶ représente hydrogène ou un groupe alkyle en C₁-C₆,
m est un entier compris entre 1 et 6,
X est choisi parmi les groupes hydroxyle, alcoxy, amino, N-alkylamino, N,N-dialkylamino, carboxyle, ester carboxylique, sulfonyle, ester sulfonique ou carbamate,
n et p sont des entiers indépendants compris entre 2 et 4, tels que n ≠ p, et
o et q sont des entiers indépendants compris entre 0 et 60, tels qu'au moins l'un des deux soit différent de 0.

12. Compositions selon la revendication 11, **caractérisées en ce que** R² représente hydrogène, méthyle ou éthyle, et R³ à R⁵ représentent hydrogène, ou **en ce que** R² représente hydrogène, méthyle ou éthyle, et que deux des résidus R³ à R⁵ représentent hydrogène, et l'un des résidus R³ à R⁵ représente méthyle ou éthyle.

13. Compositions selon au moins une des revendications 11 ou 12, **caractérisées en ce que** le poly(oxazoline) hydrosoluble comprend au moins 90 % en poids, par rapport à sa masse totale, d'unités structurales répétitives de formule I, R² représentant un groupe méthyle ou éthyle.

14. Compositions selon au moins une des revendications 1 à 13, **caractérisées en ce que** les particules de polymère organique contiennent un ou plusieurs principes actifs pharmaceutiques.

15. Utilisation de poly(oxazolines) hydrosolubles pour la stabilisation de particules de polymère organique choisies parmi les polyesters dérivés d'acides dicarboxyliques organiques et de diols organiques et/ou d'acides hydroxycarboxyliques organiques, les polycarbonates, les polymères dérivés d'esters d'acides éthylènecarboxyliques insaturés, ou d'un mélange de deux ou plusieurs de ces polymères dans un liquide hydrophile.

16. Utilisation de poly(oxazolines) hydrosolubles pour la stabilisation de particules de polymères organiques choisies parmi les polyesters dérivés d'acides dicarboxyliques organiques et de diols organiques et/ou d'acides hydroxycarboxyliques organiques, les polycarbonates, les polymères dérivés d'esters d'acides éthylènecarboxyliques insaturés, ou d'un mélange de deux ou plusieurs de ces polymères lors de la lyophilisation.

17. Utilisation de poly(oxazolines) hydrosolubles comme stabilisants lors de la production ou de la transformation de particules de polymères organiques choisies parmi les polyesters dérivés d'acides dicarboxyliques organiques et de diols organiques et/ou d'acides hydroxycarboxyliques organiques, les polycarbonates, les polymères dérivés d'esters d'acides éthylènecarboxyliques insaturés, ou d'un mélange de deux ou plusieurs de ces polymères dans un liquide hydrophile.

18. Utilisation de poly(oxazolines) hydrosolubles pour stabiliser des particules de polymères organiques en poudre sélectionnées parmi le groupe des polyesters dérivés d'acides dicarboxyliques organiques et de diols organiques et/ou d'acides hydroxycarboxyliques organiques, des polycarbonates, des polymères dérivés d'esters d'acides carboxyliques éthylène insaturés ou d'un mélange de deux ou plusieurs de ces polymères.
